# EUROPEAN PATENT APPLICATION

(11) **EP 1 967 189 A1**
(43) Date of publication of application: **10.09.2008**
(21) Application number: 08102968.8
(22) Date of filing: 18.02.2005
(51) Int. Cl.: A61K 31/282, A61P 35/00, A61K 33/24

(54) **Methods for treating resistant or refractory tumors**

(30) Priority: 18.02.2004 US 546097 P
(62) Divisional of application: 05715410.6
(71) Applicant: GPC Biotech AG, 82152 Martinsried (DE)
(72) Inventor: Caligiuri, Maureen, Reading, MA 01867 (US); Wosikowski-Buters, Katja, 85586, Poing (DE); Casazza, Anna Maria, Madison, CT 06443 (US)
(74) Representative: Dörries, Hans Ulrich

(57) **Abstract**

The instant invention relates to methods, pharmaceutical compositions and packaged pharmaceuticals for treating resistant or refractory tumors by administering platinum-based compounds.

## Description

### BACKGROUND OF THE INVENTION

Clinical drug resistance is a major barrier to overcome before chemotherapy can become curative for most patients with cancer. In many common cancers (for example, non-small cell lung, testicular and ovarian cancers), substantial tumour shrinkage can be expected in more than 50% of cases with conventional chemotherapy. In other cases, response rates are lower; only 10-20% of patients with renal cell carcinoma, pancreatic and oesophageal cancers respond to treatment. In almost all cases, drug resistance eventually develops shortly and is often fatal. If this could be treated, prevented or surmounted, the impact would be substantial.

Clinical tumour resistance to chemotherapy can be intrinsic or acquired. Intrinsic resistance is present at the time of diagnosis in tumours that fail to respond to first-line chemotherapy. Acquired resistance occurs in tumours that are often highly responsive to initial treatment, but develop resistance in the course of treatment, or on tumour recurrence, exhibiting an entirely different phenotype. They may become resistant to both previously used drugs, and new agents with different structures and mechanisms of action.

Platinum compounds are among the most active chemotherapeutic agent available for the treatment of a variety of malignancies, including testicular and ovarian carcinoma. The use of some of these compounds, e.g., cisplatin, is restricted by both toxological and resistance considerations. To overcome these issues, efforts were started to discover novel platinum compounds which do not share these properties of cisplatin. One compound that was identified is satraplatin (JM216), a platinum (Pt) IV complex. Satraplatin has advantages compared to cisplatin due to its oral availability and favourable safety profile, such as the absence of kidney- and neurotoxicity. Activity of satraplatin has been shown in prostate, ovarian and SCL carcinoma patients. In a Phase II-III clinical trial in Hormone Refractory Prostate Carcinoma (HRPC) patients, the combination of satraplatin plus prednisone was more active than prednisone alone (ASCO, 2003). The current standard treatment of HRPC is primarily palliative and includes 1^{st} line chemotherapeutic regimens with agents such as estramustine, mitoxantrone and taxanes, with docetaxol being increasingly used as a first-line chemotherapeutic agent.

Given the prevalence of clinical cancer drug resistance and its dire consequences for cancer patients, it is desirable to have methods for treating tumors resistant to a first-line therapeutic agent. Such methods are provided herein.

### SUMMARY OF THE INVENTION

The present invention provides methods, pharmaceutical compositions and packaged pharmaceuticals for treating tumors resistant or refractory to non-platinum-based therapeutic agents. Generally, the methods comprise administering an effective amount of a platinum-based compound to an individual with a cancer or tumor resistant or refractory to a non-platinum-based therapeutic agent. The platinum-based compound can be one of the following (collectively referred as the "subject platinum-based compounds"):
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 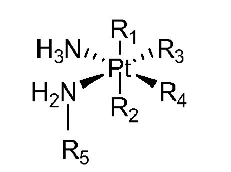 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of any of (a) to (d).

In certain preferred embodiments, the platinum-based compound is satraplatin (JM216), JM118 or JM383, or a pharmaceutically acceptable salt, isomer or prodrug thereof.

In certain embodiments, tumors resistant to non-platinum-based therapeutic agents include those for which resistance is mediated by multidrug resistance. Such multidrug resistance may be mediated through an ATP-binding cassette (ABC) transporter such as P-glycoprotein (P-gp). Non-platinum-based therapeutic agents for which resistance is mediated through P-gp include: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide) and anti-mitotics.

In other embodiments, tumors resistant to non-platinum-based therapeutic agents include those for which resistance is mediated through tubulin. Non-platinum-based therapeutic agents for which resistance is mediated through tubulin include: taxanes (paclitaxel, docetaxel, and derivatives thereof), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole.

In other embodiments, tumors resistant to non-platinum-based therapeutic agents include those for which resistance is mediated through Topoisomerase I. Non-platinum-based therapeutic agents for which resistance is mediated through Topoisomerase I include: camptothecin, 9-nitrocamptothecin (Orethecin, rubitecan), 9-aminocamptothecin (IDEC-13'), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D and irinotecan (Camptosar, CPT-11).

In yet other embodiments, tumours refractory to or previously treated with non-platinum-based therapeutic agents are suitable for practicing the methods or using the pharmaceutical compositions of the invention.

Another aspect of the invention provides methods, using the subject platinum-based compounds, for the killing or inhibiting the growth of tumor cells that are resistant or refractory to non-platinum-based therapeutic agents including those above.

Yet another aspect of the invention provides a packaged pharmaceutical comprising a subject platinum-based compound in a form suitable for use in human patients, and associated with instructions and/or a label instructing appropriate use and side effects of the subject platinum-based compound in the treatment of a tumor resistant to a non-platinum-based therapeutic agent.

Still another aspect of the invention provides a pharmaceutical composition and packaged pharmaceutical for treating tumors resistant to non-platinum-based therapeutic agents. The pharmaceutical composition and packaged pharmaceutical comprises a subject platinum-based compound.

### BRIEF DESCRIPTION OF THE DRAWING

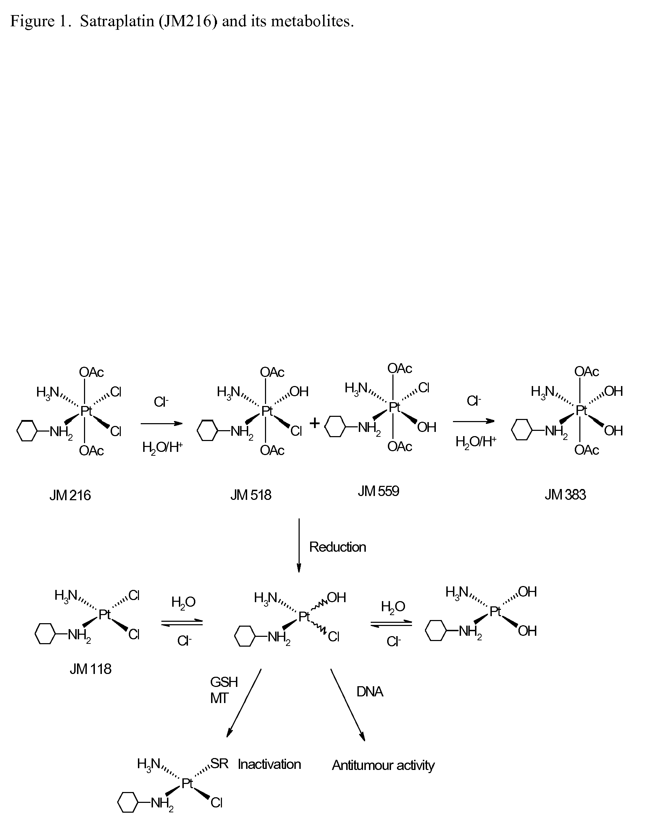

Figure 1. Satraplatin (JM216) and certain of its metabolites according to Raynaud et al 1996.

### DETAILED DESCRIPTION OF THE INVENTION

### I. Overview

The instant invention features novel methods and pharmaceutical compositions for treating tumors resistant to non-platinum-based therapeutic agents. The present invention is based, at least in part, on Applicants' discovery that the effectiveness of exemplary subject platinum-containing compounds is maintained in proliferating cells such as tumor cells resistant to various chemotherapeutic drugs. Significantly, Applicants have demonstrated that these exemplary subject platinum-based compounds can overcome drug resistance mediated through multiple different mechanisms. Each of these resistance mechanisms confers tumor resistance on a variety of drugs. In light of Applicants' discovery, patients with tumors resistant to a wide array of non-platinum-based therapeutic agents and/or chemotherapeutic drugs, including anti-cancer drugs, may benefit from treatment using the methods and pharmaceutical compositions of the present invention.

Accordingly, the present invention provides methods for treating an individual with a cancer or tumor resistant or refractory to a non-platinum-based therapeutic agent comprising administering an effective amount of a platinum-based compound. In preferred embodiments, the platinum-based compound is selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 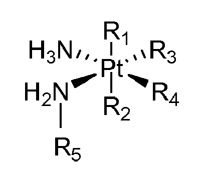 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d).

The present invention also provides methods for killing or inhibiting the growth of a tumor cell resistant to a non-platinum-based therapeutic agent comprising exposing said cell to an effective amount of a platinum-based compound. In preferred embodiments, the platinum-based compound is selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 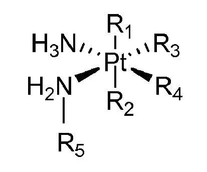 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d).

In certain embodiments, R₁ and R₂ are the same and are hydroxyl or acetate. Preferably, R₃ and R₄ are the same and are both hydroxyl or preferably halogen, such as chloride. In certain preferred embodiments, R₅ is cyclohexyl.

### II. Definitions

The terms "administered", "administration", "administering" a compound will be understood to mean providing any compound of the methods of the invention to an individual in need of treatment.

The term "alkyl" refers to optionally substituted straight- or branched-chain saturated hydrocarbon groups having from 1 to about 20 carbon atoms, preferably from 1 to about 7 carbon atoms. Examples of alkyl include, but are not limited to, methyl, ethyl, npropyl, i-propyl, n-butyl, s-butyl, t-butyl, n-pentyl, and s-pentyl. In addition, the term is intended to include both unsubstituted and substituted alkyl groups, the latter referring to alkyl moieties having one or more hydrogen substituents replaced by, but not limited to halogen, hydroxyl, carbonyl, alkoxy, ester, ether, cyano, phosphoryl, amino, imino, amido, sulfhydryl, alkythio, thioester, sulfonyl, nitro, heterocyclo, aryl or heteroaryl. It will also he understood by those skilled in the art that the substituted moieties themselves can be substituted as well when appropriate.

The term "cycloalkyl" refers to optionally substituted saturated cyclic hydrocarbon ring systems, preferably containing 3 to 7 carbons per ring. Exemplary groups include cyclopropyl, cyclobutyl, cyclopentyl, cyclohexyl, cycloheptyl, cyclooctyl, cyclodecyl, cyclododecyl, and adamantyl. Exemplary substituents include one or more alkyl groups as described above, or one or more of the groups described above as substituents for alkyl groups.

The term "effective amount" means the amount of the subject compound that will elicit the biological, physiological, pharmacological, therapeutic or medical response of a cell, tissue, system, body, animal, individual, patient or human that is being sought by the researcher, pharmacologist, pharmacist, veterinarian, medical doctor, or other clinician, e.g., lessening of the effects/symptoms of cell proliferative disorders such as a cancer or tumor, or killing or inhibiting growth of a proliferating cell, such as a tumor cell.

The term "further treated", "further administer" or "further administered", means that the different therapeutic agents may be administered together, alternatively or intermittently. Such further administration may be temporally or spatially separated, for example at different times, on different days or via different modes or routes of administration.

The term "halogen" or "halo" refers to fluorine, chlorine, bromine and iodine.

The term "IC50", as used herein, refers to concentrations at which a measurable phenotype or response, for example growth of cells such as tumor cells, is inhibited by 50%. IC50 values can be estimated from an appropriate dose-response curve, for example by eye or by using appropriate curve fitting or statistical software. More accurately, IC50 values may be determined using non-linear regression analysis.

As used herein, an "individual" means a multi-cellular organism, for example an animal such as a mammal, preferably a primate. In addition to primates, such as humans, a variety of other mammals can be treated according to the method of the present invention. For example, mammals including, but not limited to, cows, sheep, goats, horses, dogs, cats, guinea pigs, rats or other bovine, ovine, equine, canine, feline, rodent or murine species can be used.

The term "metabolite", as used herein, refers to any substance produced by metabolism or by a metabolic process. Metabolism, as used herein, refers to the various physical/chemical/biochemical/phamacological reactions involved in the transformation of molecules or chemical compounds occurring in the cell, tissue, system, body, animal, individual, patient or human therein.

The term "prodrug", as used herein, refers to an agent which is converted into a pharmacologically active parent drug in vivo. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. A prodrug may be converted into the parent drug by various mechanisms, including enzymatic processes and metabolic hydrolysis. See Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999);

As used herein, a "proliferative disorder" includes a disease or disorder that affects a cellular growth, differentiation, or proliferation process. As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process includes amino acid transport and degradation and other metabolic processes of a cell. A cellular proliferation disorder may be characterized by aberrantly regulated cellular growth, proliferation, differentiation, or migration. Cellular proliferation disorders include tumorigenic diseases or disorders. As used herein, a "tumorigenic disease or disorder" includes a disease or disorder characterized by aberrantly regulated cellular growth, proliferation, differentiation, adhesion, or migration, which may result in the production of or tendency to produce tumors. As used herein, a "tumor" includes a benign or malignant mass of tissue. Examples of cellular growth or proliferation disorders include, but are not limited to, cancer, e.g., carcinoma, sarcoma, or leukemia, examples of which include, but are not limited to, colon, ovarian, lung, breast, endometrial, uterine, hepatic, gastrointestinal, prostate, and brain cancer; turnorigenesis and metastasis; skeletal dysplasia; and hematopoietic and/or myeloproliferative disorders.

### III. Platinum-based compounds

In one embodiment of the present invention, the subject platinum-based compound is an orally available platinum chemotherapeutic agent. The phrase "orally available", as used herein, means that the drug or agent has biological, physiological, pharmacological, therapeutic, medically or clinically significant activity when administered orally. Suitable orally available platinum-based therapeutic agents include: satraplatin, (JM216), JM118 and JM383 or a pharmaceutically acceptable salt, isomer or prodrug thereof, and others described in EP 0147926 and U.S. 5,072,011. However, it should be recognised that although a platinum-based chemotherapeutic agent may be orally available, such agent may also be administered through other appropriate routes, such as rectal, s.c., i.v, i.p, or i.m., which administration would still be recognised as following the teaching of the instant invention. Likewise, other platinum compounds typically administered through non-oral routes may, through appropriate formulation or chemical modification, be rendered orally available.

In another embodiment, the platinum-based compound is a platinum (IV) coordinated compound, in which the oxidation state of the platinum is +4. Examples are satraplatin (JM216), JM518, JM559, JM383, iproplatin, tetraplatin (ormaplatin), LA-12 ((OC-6-43)-bis(acetato)(1-adamantylamine)amminedichloroplatinum(N)), JM149, JM221, JM335, ZD0473 (AMD473) and the platinum (Pt) IV compounds disclosed in US 6,413,953, US 5,072,011, US 5,519,155, US 5,547,982, US 6,518,428, WO 01/76569 and WO 02/28871, & Coordination Chemistry Reviews (2002) 232, 49-67, the entirety of which are incorporated herein.

In a further embodiment, the platinum-based compound is a platinum compound of a structure represented in the following general formula (formula I):

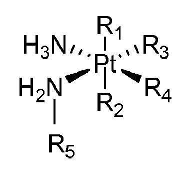

R₁-R₄ may be the same or different and are each independently selected from halogen, hydroxyl and acetate. R₅ is a cycloalkyl, preferably a cyclohexyl. In certain embodiments, R₁ and R₂ are absent. In other embodiments, R₁ and R₂ are the same and are hydroxyl or acetate. In certain embodiments, R₃ and R₄ are the same and are both hydroxyl or preferably halogen, for example, chloride.

In yet another aspect of the invention, the platinum-based chemotherapeutic agent is satraplatin, or a metabolite of satraplatin. Satraplatin (JM216) has the structure:

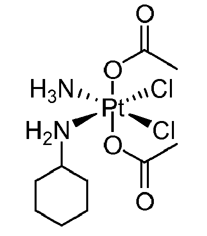

Satraplatin can be synthesised according to the method disclosed in U.S. Patent No. 5,072,011 and 5,244,919 or by appropriate modification of the method disclosed in US 6,518,428.

Upon administration of satraplatin to a cell, animal or a human patient, a number of related platinum-containing metabolites may be formed. The term "metabolite", as used herein, also includes a substance derived from a drug by physical, chemical, biological or biochemical processes in the body or cell after the drug is administered, Figure 1 (taken from Raynaud et al. 1996 Cancer Chemother Phamacol 38: 155-162) shows exemplary metabolites of satraplatin (JM216), and depicts JM118, JM383, JM518, JM559 and JM149. As will be appreciated by a person skilled in the art, additional platinum-containing molecules may be formed by metabolism of satraplatin after administration to a cell, animal or human patient, and such metabolites of satraplatin are encompassed in the scope of the instant invention. Suitable metabolites may be formed within the treated cell, animal or human by biological or biochemical biotransformation. Alternatively, such metabolites may be first formed out of the treated cell (such as in the GI tract), or may be formed by synthetic reaction from suitable starting materials and administered directly to the cell, animal or human patient. For example, JM118 may be synthesised according to the method disclosed in EP 147926, GB 2,060,615 and U.S. 4,329,299, or may be formed by biotransformation from JM216 in a separate fermentation step.

Satraplatin is considerably different from cisplatin due to its oral availability and favourable safety profile, such as the absence of kidney- and neurotoxicity. In addition, there is no cross resistance between satraplatin and cisplatin (Cancer Res, 53, 2581; Br J Cancer 68, 240). Indeed, herein we demonstrate that the efficacy of satraplatin and its metabolite is maintained in cisplatin-resistant tumor cells (Example 4).

In a preferred embodiment, the platinum-based compound is selected from satraplatin (JM216), JM118 and JM383 or a prodrug thereof. The term "prodrug", as used herein, also includes a substance that can give rise to a pharmacologically active metabolite. The prodrug itself may or may not be active; for example, it may be an inactive precursor.

An exemplary subject platinum-based chemotherapeutic agent may be administered directly to the cell, animal or human patient. However, as will be evident from the discussion of metabolites, a first platinum-based compound may be administered to a cell, following which an exemplary platinum-based chemotherapeutic agent may be formed by metabolism of the first platinum-based compound so administered. Such first platinum-based compound so administered may be considered a 'prodrug' of the exemplary subject platinum-based chemotherapeutic agent. For example, JM518 may be considered a prodrug of JM118, as JM118 (an exemplary compound useful for the method of the invention) is formed by metabolism of JM518. Analogously, JM216 may also be considered a prodrug of JM118. Other compounds that when administered to a cell, animal, individual, patient or human, are converted (metabolised) to an exemplary compound useful for the methods of the invention, such as JM118, would be considered within the scope of the instant invention. Such other compounds, may include salts, esters or phosphates of the exemplary subject compound useful for the method of the invention, and following the disclosure of the instant invention, a person skilled in the art would be able to envision a number of appropriate such prodrug compounds.

In another embodiment, the platinum-based compound is an intermediate in the synthesis of satraplatin (JM216), JM118 and JM383. Exemplary intermediates include IP-118 (U.S. Patent No. 4,687,780), JM-118 (an intermediate for synthesizing satraplatin, EP 147926) and JM149 (EP 333351).

In yet another embodiment, the platinum-based compound is represented by one of the following general structures:
(A) Those disclosed in US 5,072,011, represented by the following general structure:
   1. A Pt(IV) anti-tumor complex of the formula 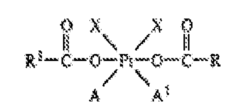 wherein A and A¹ are individually selected from the group consisting of NH₃ and an amino group of 1 to 10 carbon atoms, with the proviso that when both A and A¹ are amino groups, at least one is as amino group of 1 to 3 carbon atoms; both X groups are the same and are Cl or Br; R and R¹ are individually selected from the group consisting of C₁-C₁₀ alkyl, cycloalkyl, aryl, aralkyl of 3 to 7 carbon atoms, alkoxy, alkenyl, alkylamino of 1 to 6 carbon atoms wherein the group is joined to the carbonyl through the hetero-atom in the case of alkoxy and alkylamino, and H; such that the X groups are cis to each other and the CO₂R and CO₂R¹ groups are trans to each other.
(B) Those disclosed in US 5,244,919, represented by the following general structure: 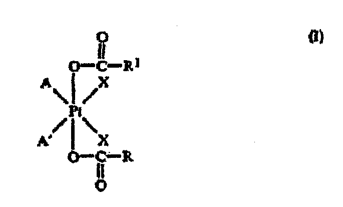 wherein A and A' are selected from the group consisting of NH_{*NH*3} and an amino group; R and R¹ are hydrogen, C₁-C₁₀ alkyl, alkenyl, aryl, aralkyl, alkylamino or alkoxy; and X is halogen or alkyl monocarboxylate or dicarboxylate.
(C) Those disclosed in US 5,519,155, as represented by the general structure:
   1. A Pt(IV) complex of general formula I, 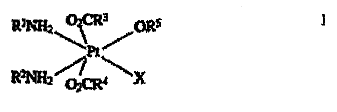 in which
      X is a halide atom, a pseudohalide, or hydroxy group,
      R¹ and R² are hydrogen, C₁ to C₆ straight or branched chain alkyl or cyclo-alkyl, aryl or R¹NH₂ is a heterocyclic nitrogen donor, and R¹ and R² may be the same as or different from one another,
      R³ and R⁴ are hydrogen, C₁ to C₅ straight or branched chain alkyl or cyclo-alkyl or aryl, and R³ and R⁴ may be the same as or different from one another, and p1 R⁵ is hydrogen, methyl or ethyl,
      and having the cis, trans, cis structure.
(D) Those disclosed EP 0 147 926 Al, as represented by the general structure: 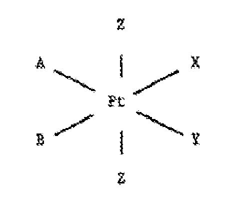 in which A and B are the same or different and are each selected from amine and alkylamines or together represent a diaminocycloalkane, X and Y are the same or different and are selected from halide and pseudohalide or together represent cycloalkanedicarboxylate, with the provisos that when X and Y together represent cycloalkanedicarboxylate A and B do not represent amine and/or alkylamine, when A and B together represent a diaminocyclohexane K and Y do not represent halide and/or pseudohalide, ans when A represents amine B does not represent ethylamine, isopropylamine or cyclopentylamine and the Z moieties are optional and are selected from halide and hydroxy, in
(E) Those disclosed in US 5,547,982 as represented by the general structures: 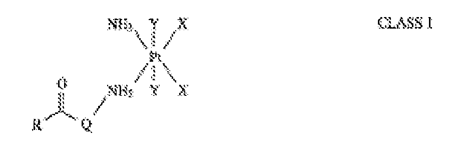 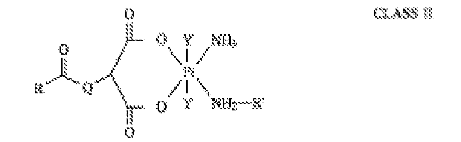 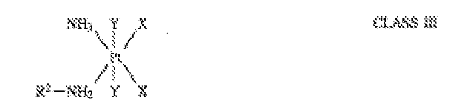 wherein R is H, lower alkyl of up to 8 carbons, alkenyl or alkynyl of up to 8 carbons or aryl; x is Cl, malonate, glycolate or oxalate Y is OH, Cl, COOR Lens B, or absent; Q is an alkylene, alkenyl, alkynyl or aryl linking group; R' is H, lower alkyl or aryl R' is H, aliphatic, aromatic or cyclo aliphatic group and R² is a cyclic aliphatic ketone, keul, bemiacetal or acetal.
(F) Those disclosed in EB 0 727 430B1 as represented by the general structures: 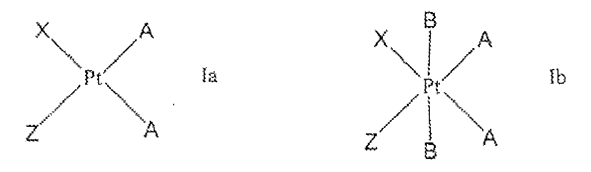 where
   each A is a leaving group and may be the same or different or together form a bi-detente carboxylate or sulphate.
   each B, which may be the same or different, is halo, hydroxy carboxylate, carbamate or carbonate ester.
   Z is a subsituted amine wherein the substituent sterically binders access of the Pt atom to a DNA strand of a tumor cell, wherein Z is an unsaturated cyclic amine coordinated to Pt through the amine nitrogen atom, which cyclic amine may contain one or more other heteroatoms and wherein said Z has a substituent on the atom adjacent the amine nitrogen atom and X is NH₃.
(G) Those disclosed in US 4,329,299 as represented by the general structures: 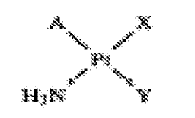 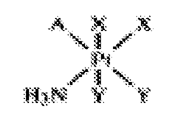 in which A is an amine having the formula R-NH₂ where R is branched chain alkyl, and X and Y are the same or different halogen.

The platinum-based compounds described above will be collectively referred herein as the "subject platinum-based compounds". The subject platinum-based compounds also encompass any such compounds in pharmaceutically acceptable salt forms. The subject platinum-based compounds of the invention may contain one or more asymmetric centers, preferably carbon or platinum, and thus occur as geometrical isomers or stereoisomers. The present invention encompasses all these isomers and mixtures thereof, as well as pharmaceutically acceptable salts and prodrugs or the subject platinum-based compounds.

### IV. Non-platinum-basedtherapeutic agent

Cancers or tumors that are resistant or refractory to treatment of a variety of therapeutic agents may benefit from treatment with the methods of the present invention. Preferred tumors are those resistant or rerfractory to non-platinum-based chemotherapeutic agents. In certain alternative embodiments of the instant invention, the subject platinum-based compounds may be useful in treating tumors that are refractory to other platinum-based chemotherapeutic agents, including cispalatin, oxaliplatin, carboplatin. For example, Example 4 demonstrates the effectiveness of certain subject platinum-based compounds, such as satraplatin (JM216) in cisplatin-resistant cells. Resistance to these platinum-based compounds can be tested and verified using the methods described in the Examples.

In preferred embodiments said non-platinum-based therapeutic agent is not a hormone based drug. In certain embodiments said non-platinum-based therapeutic agent is not a pituitary down-regulator. In other embodiments said non-platinum-based therapeutic agent is not an anti-androgen.

The term "hormone-based drugs" refers to compounds which are used in hormonal treatment. Such compounds may be hormones or derivaties or variants of hormones. Hormone-based drugs also include molecules which are neither hormones, nor derivaties or variants of hormones, yet affect the production or action of hormones. Treatment with hormone-based drugs is referred to as "hormone ablation therapy". Hormone ablation therapy aims at limiting the growth of a cancer or tumor by limiting the supply of hormones that this type of cancer or tumor needs for growth.

Some types of cancer, e.g. cancer of the prostate, depend on hormones, e.g. testosterone, for growth. If the amount of testosterone is reduced it is often possible to slow down or shrink the tumour. Such treatment is usually effective for a limited time, typically for 18 to 24 months. After that, the tumor may stop responding to the treatment and resume growth, i.e. hormone refractory prostate cancer (HRPC) develops.

Testosterone levels can be reduced, for example, by surgery (e.g. removel of the testes) or by drug-based treatment, including hormone-based drug treatment. There are two main types of such hormone based drugs. First, pituitary down-regulators block luteinizing hormone-releasing hormone (LHRH), which is released by the pituitary gland. LHRH, if not blocked is a stimulus for the testes to produce testosterone. Examples of such pituitary down-regulators include leuprorelin (Prostap), triptorelin (De-capaptyl), buserelin (Suprefact) and goserelin (Zoladex). Second, anti-androgens block the action of testosterone at the prostate. Examples of such anti-androgens include cyproterone acetate (Cyprostat), flutamide (Eulexin, Drogenil), nilutamide (Nilandrone) and bicalutamide (Casodex). It will be appreciated that other types of cancer may also be treated with hormone-based drugs. These include, but is not limited to, breast cancer, uterine cancer, thyroid cancer and colon cancer.

Suitable non-platinum-based agents for which the subject platinum-based compounds are not cross-resistant are described in the following, which may be taken as non-limiting examples of "anti-cancer therapeutic agents".

### 1. Taxanes

Resistance to taxanes like pacitaxel and docetaxol is a major problem for all chemotherapeutic regimens utilizing these drugs. Taxanes exert their cytotoxic effect by binding to tubulin, thereby causing the formation of unusually stable microtubules. The ensuing mitotic arrest triggers the mitotic spindle checkpoint and results in apoptosis. Other mechanisms that mediate apoptosis through pathways independent of microtubule dysfunction have been described as well, including molecular events triggered by the activation of Cell Division Control-2 (cdc-2) Kinase, phosphorylation of BCL-2 and the induction of interleukin 1β (IL-1β) and tumor necrosis factor-α (TNF-α). Furthermore, taxanes have been shown to also exert anti-tumor activity via mechanisms other than the direct activation of the apoptotic cascade. These mechanisms include decreased production of metalloproteinases and the inhibition of endothelial cell proliferation and motility, with consequent inhibition of angiogenesis.

As shown in the Examples, Applicants have demonstrated that exemplary subject platinum-based compounds, including satraplatin (JM216), maintain their therapeutic efficacy in tumor cell lines resistant to taxanes. In other words, tumor cell lines that are resistant to taxane treatment do not show resistance to treatment with the exemplary subject platinum-based compounds of the present invention. Thus, one embodiment of the present invention relates to methods of treating patients with tumors resistant to taxanes by administering a subject platinum-based compound of the present invention, preferably satraplatin (JM216).

By the term "taxane", it is meant to include any member of the family of terpenes, including, but not limited to paclitaxel (Taxol) and docetaxel (Taxotere), which were derived primarily from the Pacific yew tree, *Taxus_brevifolia,* and which have activity against certain tumors, particularly breast, lung and ovarian tumors (See, for example, Pazdur et al. Cancer Treat Res. 1993.19:3 5 1; Bissery et al. Cancer Res. 1991 51:4845). In the methods and packaged pharmaceuticals of the present invention, preferred taxanes are paclitaxel, docetaxel, deoxygenated paclitaxel, TL-139 and their derivatives. See Annu. Rev. Med. 48:353-374 (1997).

The term "taxane" as used herein includes both naturally derived and related forms and chemically synthesized terpenes or derivatives thereof, including deoxygenated paclitaxel compounds such as those described in U.S. Pat. Nos. 5,440,056 and 4,942,184, which are herein incorporated by reference, and that sold as TAXOL^{®} by Bristol-Myers Oncology. Pactitaxel has been approved for clinical use in the treatment of refractory ovarian cancer in the United States (Markman et al., Yale Journal of Biology and Medicine, 64:583, 1991; McGuire et al., Ann. Intern. Med., 111:273, 1989). It is effective for chemotherapy for several types of neoplasms including breast (Holmes et al., J. Nat. Cancer Inst., 83:1797, 1991) and has been approved for treatment of breast cancer as well. It is a potential candidate for treatment of neoplasms in the skin (Einzig et al., Proc. Am. Soc. Clin. Oncol., 20:46) and head and neck carcinomas (Forastire et al. Sem. Oncol., 20:56, 1990). The compound also shows potential for the treatment of polycystic kidney disease (Woo et al, Nature, 368:750, 1994), lung cancer and malaria. Docetaxel (N-debenzoyl-N-tert-butoxycarbonyl-10-deacetyl paclitaxel) is produced under the trademark TAXOTERE® by Rhone-Poulenc Rorer S.A. In addition, other taxanes are described in "Synthesis and Anticancer Activity of Taxol other Derivatives," D. G. 1. Kingston et al., Studies in Organic Chemistry, vol. 26, entitled "New Trends in Natural Products Chemistry" (1986), Atta-urRabman, P. W. le Quesne, Eds. (Elvesier, Amsterdam 1986), pp 219-235 are incorporated herein. Various taxanes are also described in U.S. Patent No. 6,380,405, the entirety of which is incorporated herein.

Methods and packaged pharmaceuticals of the present invention are applicable for treating tumors resistant to treatment by any taxane, regardless of the resistance mechanism. Known mechanisms that confer taxane resistance include, for example, molecular changes in the target molecules molecules, i.e., α-tublin and/or β-tubulin, up-regulation of P-glycoprotein (multidrug resistance gene MDR-1), changes in apoptotic regulatory and mitosis checkpoint proteins, changes in cell membranes, overexpression of interleukin 6 (IL-6; Clin Cancer Res (1999) 5, 3445-3453; Cytokine (2002) 17, 234-242), the overexpression of interleukin 8 (IL-8; Clin Cancer Res (1999) 5, 3445-3453; Cancer Res (1996) 56, 1303-1308) or the overexpression of monocyte chemotactic protein-1 (MCP-1; (MCP-1; Clin Cancer Res (1999) 5, 3445-3453), changes in the levels of acidic and basic fibroblast growth factors, transmembrane factors, such as p185 (HER2; Oncogene (1996) 13, 1359-1365) or EGFR (Oncogene (2000) 19, 6550-6565; Bioessays (2000) 22, 673-680), changes in adhesion molecules, such as β1 integrin (Oncogene (2001) 20, 4995-5004), changes in house keeping molecules, such as glutathione-S-transferase ans/or glutathione peroxidase (Jpn J Clin Oncol (1996) 26, 1-5), changes in molecules involved in cell signalling, such as interferone response factor 9, molecules involved in NF-κB signalling, molecules involved in the PI-3 kinase/AKT survival pathway, RAF-1 kinase activity, PKC α/β or PKC β/β2 and via nuclear proteins, such as nuclear annexin IV, the methylation controlled J protein of the DNA J family of proteins, thymidylate synthetase or c-jun.

Another known mechanism that confers taxane resistance is, for example, changes in apoptotic regulatory and mitosis checkpoint proteins. Such changes in apoptotic regulatory and mitosis checkpoint proteins include the overexpresion of Bcl-2(Cancer Chemother Pharmacol (2000) 46, 329-337; Leukemia (1997) 11, 253-257) and the overexpresion of Bcl-xL (Cancer Res (1997) 57, 1109-1115; Leukemia (1997) 11, 253-257). Overexpresion of Bcl-2 may be effected by estradiol (Breast Cancer Res Treat (1997) 42, 73-81).

Taxane resistance may also be conferred via changes in the cell membrane. Such changes include the change of the ratio of fatty acid methylene:methyl (CancerRes (1996) 56, 3461-3467), the change of the ratio of choline:methyl(CancerRes (1996)56, 3461-3467) and a change of the permeability of the cell membrane (J Cell Biol (1986) 102, 1522-1531).

Further known mechanisms that confer taxane resistance are changes in acidic and basic fibroblast growth factors (Proc Natl Acad Sci USA (2000) 97, 8658-8663), molecules involved in cell signalling, such as interferone response factor 9 (Cancer Res (2001) 61, 6540-6547), molecules involved in NF-κB signalling (Surgery (2991) 130, 143-150), molecules involved in the PI-3 kinase/AKT survival pathway (Oncogene (2001) 20, 4995-5004), RAF-1 kinase activity (Anticancer Drugs (2000) 11, 439-443; Chemotherapy (2000) 46, 327-334), PKC α/β (Int J Cancer (1993) 54" 302-308) or PKC β/β2 (Int J Cancer (2001) 93, 179-184, Anticancer Drugs (1997) 8, 189-198).

Taxane resistance may also be conferred via changes nuclear proteins, such as nuclear annexin IV (Br J Cancer (2000) 83, 83-88), the methylation controlled J protein of the DNA J family of proteins (Cancer Res (2001) 61, 4258-4265), thymidylate synthetase (Anticancer Drugs (1997) 8, 189-198) or c-jun (Anticancer Drugs (1997) 8, 189-198), via paracrine factors, such as LPS (J Leukoc Biol (1996) 59, 280-286), HIF-1 (Mech Dev (1998) 73, 117-123), VEGF (Mech Dev (1998) 73, 117-123) and the lack of decline in bcl-XL in spheroid cultures (Cancer Res (1997) 57, 2388-2393).

### 2. Indole Alkaloids

As shown in the Examples, Applicants have demonstrated that exemplary subject platinum-based compounds maintain its therapeutic efficacy in tumors resistant to camptothecin, an indole alkaloid. In other words, tumors that are resistant to camptothecin treatment do not show resistance to treatment with the exemplary subject platinum-based compounds of the present invention. Thus, one embodiment of the present invention relates to methods of treating patients with tumors resistant to an indole alkaloid by administering a platinum-based compound of the present invention.

Exemplary indole alkaloids include bis-indole alkaloids, such as vincristine, vinblastine and 5'-nor-anhydrovinblastine (hereinafter: 5'-nor- vinblastine). It is known that bis-indole compounds (alkaloids), and particularly vincristine and vinblastine of natural origin as well as the recently synthetically prepared 5'-nor-vinblastine play an important role in the antitumour therapy. These compounds were commercialized or described, respectively in the various pharmacopoeias as salts (mainly as sulfates or difumarates, respectively).

Preferred indole alkaloids are camptothecin and its derivatives and analogues. Camptothecin is a plant alkaloid found in wood, bark, and fruit of the Asian tree Camptotheca acuminata. Camptothecin derivatives are now standard components in the treatment of several malignancies. See Pizzolato and Saltz, 2003. Studies have established that Camptothecin inhibited both DNA and RNA synthesis. Recent research has demonstrated that Camptothecin and its analogs interfere with the mechanism of action of the cellular enzyme topoisomerase I, which is important in a number of cellular processes (e.g., DNA replication and recombination, RNA transcription, chromosome decondensation, etc.). Without being bound to theory, camptothecin is thought to reversibly induce single-strand breaks, thereby affecting the cell's capacity to replicate. Camptothecin stabilises the so-called cleavable complex between topoisomerase I and DNA. These stabilized breaks are fully reversible and non-lethal. However, when a DNA replication fork collides with the cleavable complex, single-strand breaks are converted to irreversible double-strand breaks. Apoptotic cell death is then mediated by caspase activation. Inhibition of caspase activation shifts the cells from apoptosis to transient G1 arrest followed by cell necrosis. Thus, the mechanisms of cell death need active DNA replication to be happening, resulting in cytotoxic effects from camptothecin that is S-phase-specific. Indeed, cells in S-phase in vitro have been shown to be 100-1000 times more sensitive to camptothecin than cells in G1 or G2.

Camptothecin analogues and derivatives include, for example, irinotecan (Camptosar, CPT-11), topotecan (Hycamptin), BAY 38-3441, 9-nitrocamptothecin (Orethecin, rubitecan), exatecan (DX-8951), lurtotecan (GI-147211C), gimatecan, homocamptothecins diflomotecan (BN-80915) and 9-aminocamptothecin (IDEC-13'). See Pizzolato and Saltz, The Lancet, 361:2235-42 (2003); and Ulukan and Swaan, Drug 62: 2039-57 (2002). Additional camptothecin analogues and derivatives include SN-38 (the active compound of the prodrug irinotecan; conversion is catalyzed by cellular carboxylesterases), ST1481, karanitecin (BNP1350), indolocarbazoles, such as NB-506, protoberberines, intoplicines, idenoisoquinolones, benzo-phenazines and NB-506

The methods and pharmaceutical compositions of the present invention are useful for treating tumors resistant to any one or more of above-listed drugs. More camptothecin derivatives are described in WO 03/101998; US Patent No. 6100273: and, US Patent No. 5587673,.

### 3. Other non-platinum-based therapeutic agents

Applicants have demonstrated that the subject platinum-based agents are effective in treating resistant tumors in which resistance is mediated through at least one of the following three mechanisms: multidrug resistance, tubulins and topoisomerase I. This section describes these three resistance mechanisms and therapeutic agents for which resistance arises through at least one of these mechanisms. One of skill in the art will understand that tumor cells may be resistant to a chemotherapeutic agent through more than one mechanism. For example, the resistance of tumor cells to paclitaxel may be mediated through via multidrug resistance, or alternatively or additionally, via tubulin mutation(s).

In a preferred embodiment, the methods and pharmaceutical compositions of the present invention are useful for treating tumors resistant to non-platinum-based chemotherapeutic agents.

In an alternative embodiment, the methods, packaged pharmaceuticals and pharmaceutical compositions of the present invention are useful for treating tumors resistant to platinum-based chemotherapeutic agents.

### a. Resistance mediated through tubulins

Microtubules are intracellular filamentous structures present in all eukaryotic cells. As components of different organelles such as mitotic spindles, centrioles, basal bodies, cilia, flagella, axopodia and the cytoskeleton, microtubules are involved in many cellular functions including chromosome movement during mitosis, cell motility, organelle transport, cytokinesis, cell plate formation, maintenance of cell shape and orientation of cell microfibril deposition in developing plant cell walls. The major component of microtubules is tubulin, a protein composed of two subunits called alpha and beta. An important property of tubulin in cells is the ability to undergo polymerization to form microtubules or to depolymerize under appropriate conditions. This process can also occur in vitro using isolated tubulin.

Microtubules play a critical role in cell division as components of the mitotic spindle, an organelle which is involved in distributing chromosomes within the dividing cell precisely between the two daughter nuclei. Various drugs prevent cell division by binding to tubulin or to microtubules. Anticancer drugs acting by this mechanism include the alkaloids vincristine and vinblastine, and the taxane-based compounds paclitaxel and docetaxel {see, for example, E. K. Rowinsky and R. C. Donehower, Pharmacology and Therapeutics, 52, 35-84 (1991)}. Other antitubulin compounds active against mammalian cells include benzimidazoles such as nocodazole and natural products such as colchicine, podophyllotoxin, epithilones, and the combretastatins.

Non-platinum-based therapeutic agents may exert their activities by, for example, binding to α-tubulin, β-tubulin or both, and/or stabilizing microtubules by preventing their depolymerization. Other modes of activity may include, down regulation of the expression of such tubulin proteins, or binding to and modification of the activity of other proteins involved in the control of expression, activity or function of tubulin.

In one embodiment, the resistance of tumor cells to a non-platinum-based therapeutic agent is mediated through tubulin. By "mediated through tubulin", it is meant to include direct and indirect involvement of tubulin. For example, resistance may arise due to tubulin mutation, a direct involvement of tubulin in the resistance. Alternatively, resistance may arise due to alterations elsewhere in the cell that affect tubulin and/or microtubules. These alterations may be mutations in genes affecting the expression level or pattern of tubulin, or mutations in genes affecting microtubule assembly in general. Mammals express 6 α- and 6 β-tubulin genes, each of which may mediate drug resistance.

Specifically, tubulin-mediated tumor resistance to non-platinum-based therapeutic agents may be conferred via molecular changes in the tubulin molecules. For example, molecular changes include mutations, such as point mutations, deletions or insertions, splice variants or other changes at the gene, message or protein level. In particular embodiments, such molecular changes may reside in amino acids 250-300 of β-tubulin, or may effect nucleotides 810 and/or 1092 of the β-tubulin gene. For example, and without wishing to be limited, the paclitaxel-resistant human ovarian carcinoma cell line 1A9-PTX10 is mutated at amino acid residues β 270 and β 364 of β-tubulin (see Giannakakou et al., 1997). For another example, two epothilone-resistant human cancer cell lines has acquired β-tubulin mutations at amino acid residues β274 and β282, respectively (See Giannakakou et al., 2000). These mutations are thought to affect the binding of the drugs to tubulins. Alternatively, mutations in tubulins that confer drug resistance may also be alterations that affect microtubule assembly. This change in microtubule assembly has been demonstrated to compensate for the effect of drugs by having diminished microtubule assembly compared to wild-type controls (Minotti, A. M., Barlow, S. B., and Cabral, F. (1991) J Biol Chem 266, 3987-3994). It will also be understood by a person skilled in the art that molecular changes in α-tubulin may also confer resistance to certain compounds. WO 00/71752 describes a wide range of molecular changes to tubulin molecules and the resistance to certain chemotherapeutic compounds that such molecular changes may confer on a cell. WO 00/71752, and all references therein, are incorporated in their entirety herein.

Tubulin-mediated tumor resistance to non-platinum-based therapeutic agents may also be conferred via alterations of the expression pattern of either α-tubulin or the β-tubulin, or both. For example, several laboratories have provided evidence that changes in the expression of specific β-tubulin genes are associated with paclitaxel resistance in cultured tumor cell lines (Haber, M., Burkhart, C. A., Regl, D. L., Madafiglio, J., Norris, M. D., and Horwitz, S. B. (1995) J BioL Chem. 270, 31269-75; Jaffrezou, J. P., Durnontet, C., Deny, W. B., Duran, G., Chen, G., Tsuchiya, E., Wilson, L., Jordan, M. A., and Sikic, B. 1. (1995) Oncology Res. 7, 517-27; Kavallaris, M., Kuo, D. Y. S., ., Burkhart, C. A., RegI, D. L., Norris, M. D., Haber, M., and Horwitz, S. B. (1997) J Clin.Invest. 100, 1282-93; and Ranganathan, S., Dexter, D. W., Benetatos, C. A., and Hudes, G. R. (1998) Biochi,. Biophys. Acta 1395, 237-245).

Tubulin-mediated tumor resistance to non-platinum-based therapeutic agents may also be conferred via an increase of the total tubulin content of the cell, an increase in the α-tubulin content or the expression of different electrophoretic variants of α-tubulin. Furthermore, resistance may be conferred via alterations in the electrophoretic mobility of β-tubulin subunits, overexpression of the Hβ2 tubulin gene, overexpression of the Hβ3 tubulin gene, overexpression of the Hβ4 tubulin gene, overexpression of the Hβ4a tubulin gene or overexpression of the Hβ5 tubulin gene,

Tubulin-mediated tumor resistance to non-platinum-based therapeutic agents may also be conferred via post-translational modification of tubulin, such as increased acetylation of α-tubulin (Jpn J Cancer Res (85) 290-297), via proteins that regulate microtubule dynamics by interacting with tubulin dimmers or polymerized microtubules. Such proteins include but are not limited to stathmin (Mol Cell Biol (1999) 19, 2242-2250) and MAP4 (Biochem Pharmacol (2001) 62, 1469-1480).

Exemplary chemotherapeutic agents for which resistance is at least partly mediated through tubulin include, taxanes (paclitaxel, docetaxel and derivatives thereof), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole. See WO 03/099210 and Giannakakou et al., 2000. Additional exemplary chemotherapeutic agents for which resistance is at least partly mediated through tubulin include, colchicine, curacin, combretastatins, cryptophycins, dolastatin, auristatin PHE, symplostatin 1, eleutherobin, halichondrin B, halimide, hemiasterlins, laulimalide, maytansinoids, PC-SPES, peloruside A, resveratrol, S-allylmercaptocysteine (SAMC), spongistatins, taxanes, vitilevuamide, 2-methoxyestradiol (2-ME2), A-289099, A-293620/A-318315, ABT-751/E7010, ANG 600 series, anhydrovinblastine (AVLB), AVE806, bivatuzumab mertansine, BMS-247550, BMS-310705, cantuzumab mertansine, combretastatin, combretastatin A-4 prodrug (CA4P), CP248/CP461, D-24851/D-64131, dolastatin 10, E7389, EPO906, FR182877, HMN-214, huN901-DM1/BB-10901TAP, ILX-651, KOS-862, LY355703, mebendazole, MLN591DM1, My9-6-DM1, NPI-2352 andNPI-2358, Oxi-4503, R440, SB-715992, SDX-103, T67/T607, trastuzumab-DM1, TZT-1027, vinflunine, ZD6126, ZK-EPO.

Resistance to these and other compounds can be tested and verified using the methods described in the Examples. The methods and pharmaceutical compositions of the present invention are useful for treating tumors resistant to any one or more of above-listed agents.

Preferred chemotherapeutic agents for which resistance is at least partly mediated through tubulin are taxanes, including, but not limited to paclitaxel and docetaxel (Taxotere), which were derived primarily from the Pacific yew tree, Taxus brevifolia, and which have activity against certain tumors, particularly breast and ovarian tumors (See, for example, Pazdur et al. Cancer Treat Res. 1993.19:3 5 1; Bissery et al. Cancer Res. 1991 51:4845).

### b. Resistance mediated through multidrug resistance

In another embodiment, the resistance of tumor cells to a non-platinum-based therapeutic agent is mediated through multidrug resistance. The term "multidrug resistance (MDR)", as used herein, refers to a specific mechanism that limits the ability of a broad class of hydrophobic, weakly cationic compounds to accumulate in the cell. These compounds have diverse structures and mechanisms of action, yet all are affected by this mechanism.

Experimental models demonstrate that multidrug resistance can be caused by increased expression of ATP-binding cassesette (ABC) transporters, which function as ATP-dependent efflux pumps. These pumps actively transport a wide array of anti-cancer and cytotoxic drugs out of the cell, in particular natural hydrophobic drugs. In mammals, the superfamily of ABC transporters includes P-glycoprotein (P-gp, ABCB1) transporters (MDR1 and MDR3 genes in human), the MRP subfamily (already composed of six members, e.g MRP1 (ABCC1)), and bile salt export protein (ABCB11; Cancer Res (1998) 58, 4160-4167), MDR-3 (Nature Rev Cancer (2002) 2, 48-58), lung resistance protein (LRP) and breast cancer resistant protein (BCRP, ABCG2). See Kondratov et al., 2001 and references therein; Cancer Res (1993) 53, 747-754; J Biol Chem (1995) 270, 31269-31275; Leukemia (1994) 8, 465-475; Biochem Pharmacol (1997) 53, 461-470). These proteins can recognize and efflux numerous substrates with diverged chemical structure, including many anticancer drugs. Overexpression of P-gp is the most common cause for MDR. Other causes of MDR have been attributed to changes in topoisomerase II, protein kinase C and specific glutathione transferase enzymes. See Endicott and Ling, 1989.

The methods of the present invention are useful for treating tumors resistant to a non-platinum-based therapeutic agent, in which resistance is at least partially due to MDR. In a preferred embodiment, the drug resistance of the tumor is mediated through overexpression of an ABC transporter. In a further preferred embodiment, the drug resistance of the tumor is mediated through the overexprssion of P-gp. Numerous mechanisms can lead to overexpression of P-gp, including amplification of the MDR-1 gene (Anticancer Res (2002) 22, 2199-2203), increased transcription of the MDR-1 gene (J Clin Invest (1995) 95, 2205-2214; Cancer Lett (1999) 146, 195-199; Clin Cancer Res (1999) 5, 3445-3453; Anticancer Res (2002) 22, 2199-2203), which may be mediated by transcription factors such as RGP8.5 (Nat Genet 2001 (27), 23-29), mechanisms involving changes in MDR-1 translational efficiency (Anticancer Res (2002) 22, 2199-2203), mutations in the MDR-1 gene (Cell (1988) 53, 519-529; Proc Natl Acad Sci USA (1991) 88, 7289-7293; Proc Natl Acad Sci USA (1992) 89, 4564-4568) and chromosomal rearrangements involving the MDR-1 gene and resulting in the formation of hybrid genes (J Clin Invest (1997) 99, 1947-1957).

In other embodiments, the methods of the present invention are useful for treating tumors resistant to a non-platinum-based therapeutic agent, in which resistance is due to other causes that lead to MDR, including, for example, changes in topoisomerase II, protein kinase C and specific glutathione trasnferase enzyme.

Therapeutic agents to which resistance is conferred via the action of P-gp include, but is not limited to: vinca alkaloids (e.g., vinblastine), the anthracyclines (e.g., adriamycin, doxorubicin), the epipodophyllotoxins (e.g., etoposide), taxanes (e.g., paclitaxel, docetaxel), antibiotics (e.g., actinomycin D and gramicidin D), antimicrotubule drugs (e.g., colchicine), protein synthesis inhibitors (e.g., puromycin), toxic peptides (e.g., valinomycin), topoisomerase inhibitors (e.g., topotecan), DNA intercalators (e.g., ethidium bromide) and anti-mitotics. See WO 99/20791. The methods and pharmaceutical compositions of the present invention are useful for treating tumors resistant to any one or more of above-listed drugs.

### c. Resistance mediated through topoisomerase I

In a further embodiment, the resistance of tumor cells to a non-platinum-based therapeutic agent is mediated through topoisomerase. Exemplary therapeutic agents that belong to this category include those that target topoisomerase, either directly or indirectly.

DNA normally exists as a supercoiled double helix. During replication, it unwinds, with single strands serving as a template for synthesis of new strands. To relieve the torsional stress that develops ahead of the replication fork, transient cleavage of one or both strands of DNA is needed. Without wishing to be bound to any mechanism, it is believed that topoisomerases facilitate this process as follows: Topoisomerase II causes transient double-stranded breaks, whereas topoisomerase I causes single-strand breaks. This action allows for rotation of the broken strand around the intact strand. Topoisomerase I then re-ligates the broken strand to restore integrity of doublestranded DNA.

In one embodiment, resistance of tumor cells to a non-platinum-based therapeutic agent is mediated through topoisomerase. By "mediated through topoisomerase", it is meant to include direct and indirect involvement of topoisomerase. For example, resistance may arise due to topoisomerase mutation, a direct involvement of topoisomerase in the resistance. Alternatively, resistance may arise due to alterations elsewhere in the cell that affect topoisomerase. These alterations may be mutations in genes affecting the expression level or pattern of topoisomerase, or mutations in genes affecting topoisomerase function or activity in general. In preferred embodiments, said topoisomerase is topoisomerase I. In other embodiments, said topoisomerase is Topoisomerase II.

Without being bound by theory, compounds that act on topoisomerase I bind to the topoisomerase I-DNA complex in a manner that prevents the religation of DNA. Topoisomerase I initially covalently interacts with DNA. Topoisomerase I then cleaves a single strand of DNA and forms a covalent intermediate via a phosphodiester linkage between tyrosine-273 of topoisomerase I and the 3'-phosphate group of the scissile strand of DNA. The intact strand of DNA is then passed through the break and then topoisomerase I religates the DNA and releases the complex. Drugs such as camptothecins bind to the covalent complex in a manner that prevents DNA religation. The persistent DNA breaks induce apoptosis, likely via collisions between these lesions and or replication or transcription complexes.

Preferred therapeutic agents to which resistance is mediated through topoisomerase I include camptothecin and its derivatives and analogues, such as 9-nitrocamptothecin (IDEC-132), exatecan (DX-8951f), rubitecan (9-nitrocamptothecin), lurtotecan (GI-147211C), the homocamptothecins such as diflomotecan (BN-80915) and BN-80927, topotecan, NB-506, J107088, pyrazolo [1,5-a] indole derivatives, such as GS-5, lamellarin D, SN-38, 9-aminocamptothecin, ST1481 andkaranitecin (BNP1350) and irinotecan (CPT-11). Other camptothecins that may be used to practice the instnat invention can be found in The Camptothecins: Unfolding Their Anticancer Potential, Annals of the New York Academy of Science, Volume 922 (ISBN 1-57331-291-6).

Without wishing to be bound by any particular theory, it is believed that camptothecins inhibit topoisomerase I by blocking the rejoining step of the cleavage/religation reaction of topoisomerase I, resulting in accumulation of a covalent reaction intermediate, the cleavable complex. Specifically, topoisomerase I-mediated tumor resistance to non-platinum-based therapeutic agents may be conferred via molecular changes in the topoisomerase I molecules. For example, molecular changes include mutations, such as point mutations, deletions or insertions, splice variants or other changes at the gene, message or protein level.

In particular embodiments, such molecular changes reside near the catalytic tyrosine residue at amino acid position 723. Residues at which such molecular changes may occur include but are not limited to amino acid positions 717, 722, 723, 725, 726, 727, 729, 736 and 737 (see Oncogene (2003) 22, 7296-7304 for a review).

In equally preferred embodiments, such molecular changes reside between amino acids 361 and 364. Residues at which such molecular changes may occur include but are not limited to amino acid positions 361, 363 and 364.

In other equally preferred embodiments, such molecular changes reside near amino acid 533. Residues at which such molecular changes may occur include but are not limited to amino acid positions 503 and 533.

In other equally preferred embodiments, such molecular changes may also reside in other amino acids of the topoisomerase I protein. Residues at which such molecular changes may occur include but are not limited to amino acid positions 418 and 503 .

In other embodiments, such molecular changes may be a duplication. In one embodiment such a duplication may reside the nucleotides corresponding to amino acids 20-609 of the topoisomerase I protein.

In other embodiments, topoisomerase I-mediated tumor resistance may also be conferred via cellular proteins that interact with topoisomerase-1. Proteins that are able to do so include, but are not limited to, nucleolin.

In particular embodiments, such molecular changes may reside in amino acids 370 and/or 723. For example, and without wishing to be limited, the camptothecin-resistant human leukemia cell line CEM/C2 (ATCC No. CRL-2264) carries two amino acid substitution at positions 370 (Met→Thr) and 722 (Asn→Ser) (Cancer Res (1995) 55, 1339-1346). The camptothecin resistant CEM/C2 cells were derived from the T lymphoblastoid leukemia cell line CCRF/CEM by selection in the presence of camptohecin *in vitro* (Kapoor et al., 1995. Oncology Research 7; 83-95, ATCC). The CEM/C2 resistant cells display atypical multidrug resistance and express a form of topoisomerase I that is less sensitive to the inhibitory action of camptothecin than that from CCRF/CEM cells at a reduced level relative to the parental cells. In addition to resistance to camptothecin, the CEM/C2 cells exhibit cross resistance to etoposide, dactinomycin, bleomycin, mitoxantrone, daunorubicin, doxorubicin and 4'-(9-acridinylamino)methanesulfon-m-anisidide.

In other embodiments, topoisomerase I-mediated tumor resistance to non-platinum-based therapeutic agents may also be conferred via alterations of the expression pattern the topoisomerase I gene (Oncol Res (1995) 7, 83-95). In further embodiments, topoisomerase 1-mediated tumor resistance may also be conferred via altered metabolism of the drug. In yet further embodiments, topoisomerase 1-mediated tumor resistance may also be conferred via inadequate and/or reduced accumulation of drug in the tumor, alterations in the structure or location of topoisiomerase 1, alterations in the cellular response to the topoisomerase I-drug interaction or alterations in the cellular response to drug-DNA-ternary complex formation (Oncogene (2003) 22, 7296-7304; Ann N Y Acad Sci (2000) 922, 46-55).

Topoisomerase I is believed to move rapidly from the nucleolus to the nucleus or even cytoplasm after cellular exposure to camptothecins.

In one embodiment topoisomerase I-mediated tumor resistance is mediated through factors involved in the relocation of topoisomerase I from the nucleolus to the nucleus and/or the cytoplasm, such as factors involved in the ubiquitin/26S proteasome pathway or SUMO.

In other embodiments topoisomerase I-mediated tumor resistance is mediated through factors involved in DNA replication, DNA checkpoint control and DNA repair.

Factors of the DNA checkpoint control include proteins of the S-checkpoint control, such as Chkl, ATR, ATM, and the DNA-PK multimer.

In other embodiments topoisomerase I-mediated tumor resistance is mediated via factors of apoptosis pathways or other cell death pathways. This includes, but is not limited to, the overexpression of bcl-2 and the overexpression of p21^{Waf1/Cip1}.

In other embodiments topoisomerase 1-mediated tumor resistance is mediated via post-translational modifications of topoisomerase I. Such post-translational modifications are ubiquitination and sumoylation. Furthermore, such post-translational modifications may involve other cellular proteins, such as Ubp11, DOA4 and topor.

Therapeutic agents to which resistance is mediated through topoisomerase II include epipodophyllotoxins, such as VP 16 and VM26, [1,5-a], pyrazolo [1,5-a] indole derivatives, such as GS-2, GS-3, GS-4 and GS-5.

### V. Assays for effectiveness of treatment

In one embodiment, the platinum-based compounds of the present invention kill tumor cells resistant to a non-platinum-based therapeutic agent. Viability of a tumor cell can be determined by any methods known in the art. For example, one may use the colorimetric cytotoxicity assay described for anticancer drug screening in Shekan et al., J. Natl. Cancer. Inst. 82: 1107-12 (1990). For another example, one may determine the viability of a tumor cell by contacting the cell with a dye and viewing it under a microscope. Viable cells can be observed to have an intact membrane and do not stain, whereas dying or dead cells having "leaky" membranes do stain. Incorporation of the dye by the cell indicates the death of the cell. A dye useful for this purpose is trypan blue.

The exemplary platinum-containing composition of the present invention may induce apoptosis, a mode of cell death, in resistant tumor cells. Apoptosis is recognized by a characteristic pattern of morphological, biochemical and molecular changes. Cells going through apoptosis appear shrunken and rounded. They also can be observed to become detached from a culture dish in which they are maintained. The morphological changes involve a characteristic pattern of condensation of chromatin and cytoplasm which can be readily identified by microscopy. When stained with a DNA-binding dye, e.g., H33258, apoptotic cells display classic condensed and punctuate nuclei instead of homogenous and round nuclei.

A typical characteristic of apoptosis is endonucleolysis, a molecular change in which nuclear DNA is initially degraded at the linker sections of nucleosomes to give rise to fragments equivalent to single and multiple nucleosomes. When these DNA fragments are subjected to gel electrophoresis, they reveal a series of DNA bands which are positioned approximately equally distant from each other on the gel. The size difference between the two bands next to each other is about the length of one nucleosome, i.e., 120 base pairs. This characteristic display of the DNA bands is called a DNA ladder and it indicates apoptosis of the cell. Apoptotic cells can also be identified by flow cytometric methods based on measurement of cellular DNA content, increased sensitivity of DNA to denaturation, or altered light scattering properties. These methods are well known in the art. It should be recognized however, that modes of programmed cell death, including apoptosis, may following a number of mechanisms or show other phenotypes/properties to those described above. In such cases, these mechanisms may also be characterized, classified or considered as "apoptosis".

Cytotoxicity may also be measured using the SRB assay according to Shekan et al (J Natl Cancer Inst (1990) 82, 1107-112), as described in the Examples.

Additional assays for cell viability are described in Chapter 15 of Handbook of Fluorescent Probes and Research Products (Molecular Probes Handbook), which is incorporated in its entirety herein.

In another embodiment, the platinum-based compounds of the present invention inhibit the growth of tumor cells resistant to a non-platinum-based therapeutic agent. The growth inhibition of resistant tumor cells caused by a platinum-based compound can be either partial (slowing down cell growth) or complete inhibition (i.e., arresting cells at a certain point in cell cycle). Cell growth can be measured by any techniques known in the art. Such techniques include, for example, MTT assay (based on reduction of the tetrazolium salt 3, [4,5-dimethylthiazol-2-yl]-2,5-diphenytetrazolium bromide), and PicoGreen assay using the DNA-binding dye Picogreen, both of which are described in Torrance, et al., Nat. Biotech. 19:940-945 (2001), incorporated herein in its entirety. Other assays for cell proliferation/growth are described in Chapter 15 of Handbook of Fluorescent Probes and Research Products (Molecular Probes Handbook).

Progression of disease, cancer or tumor in response to treatment using the subject platinum-based compounds can be monitored using any standard technique known in the art. For example, tumor size can be monitored and assessed to see if tumor size reduction has occurred as a result of the treatment. Monitoring and assessment may be aided by a variety of means including biopsies, manual inspection, microscopy, whole or partial body imaging and scans, and various molecular-based diagnostic and prognostic methods including those that investigate tumor-specific markers or mutations.

### VI. Tumors and Other Proliferative Disorders

The subject platinum-based compounds are useful to treat proliferative disorders resistant to a non-platinum-based therapeutic agent. The term "proliferative disorder" is also art-recognized andfurther includes a disorder affecting an animal in a manner which is marked by abberant, or otherwise unwanted, proliferation of a subset of cells of an animal. Cancer and tumors are proliferative disorders. Cells comprising or derived from a tumor will generally be understood to be a proliferating cell, typically a hyper-proliferating cell, and in other circumstances, a tumor cell may be dysplastic, or may have proliferated.

It will be apparent to a person skilled in the art, on reading the disclosure of the instant invention, that the methods, pharmaceutical compositions and packaged pharmaceuticals comprising the subject platinum-based compounds will be useful for the treatment of other proliferative disorders, or for killing or inhibiting proliferating cells including tumor cells.

Tumors that are resistant or refractory to treatment with a variety of chemotherapeutic agents may benefit from treatment with the methods and pharmaceutical compositions of the present invention. Suitable tumors may be solid tumors, which are cancer of body tissues other than blood, bone marrow, or the lymphatic system. Preferred tumors are those resistant to non-platinum-based chemotherapeutic agents. Suitable tumors may also be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, skin cancer, cervical cancer, ovarian cancer, lung cancer, bronchial, small and non-small-cell lung cancer, gastric, prostate, pancreas, and head and neck cancer.

Hematological tumors may be leukemia, such as Acute Myelogenous Leukemia (AML), Acute Lymphoblastic Leukemia (ALL), Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, and Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin Lymphoma, Non-Hodgkin Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Extramedullary myeloma, and Granulocytic Sarcomae.

In certain embodiments, the methods and compositions of the present invention can be used to treat tumors resistant or refractory to taxanes. Such tumors include, for example, breast cancer, cervical cancer, colorectal cancer, peritoneal cancer, ovarian cancer, bronchial cancer, small cell lung cancer, non-small cell lung cancer, gastric, prostate, and head and neck cancer, including recurrent squamous cell carcinomas.

In other embodiments, the methods and compositions of the present invention are applicable for treating tumors resistant or refractory to camptothecin and its analogues. Such tumors include any tumor for which camptothecin and its analogues have shown activities. Examples of such tumors include ovarian cancer and small-cell lung cancer, for which both topotecan and irinotecan have shown effectiveness, colorectal cancer, upper gastrointestinal malignancies, non-small-cell lung cancer, mesothelioma, and head and neck cancer, for which irinotecan has shown effectiveness, small-cell lung cancer, cervical cancer, breast cancer, prostate cancer, rectal cancer, leukemia, lymphoma cancers and malignant melanoma.. See Pizzolato and Saltz, The Lancet 361:2235-42 (2003).

Refractory cancers or tumors include those that fail or are resistant to treatment with chemotherapeutic agents alone, radiation alone or combinations thereof. For the purposes of this specification, refractory cancers or tumors also encompass those that appear to be inhibited by treatment with chemotherapeutic agents and/or radiation but recur up to five years, sometimes up to ten years or longer after treatment is discontinued.

The term "resistant", as used herein, include both partially resistant and completely resistant. Thus, a tumor that is only partially resistant to a non-platinum-based therapeutic agent may nonetheless benefit from treatment with the subject platinum-based compounds. Indeed, in certain embodiments it may be beneficial to treat a tumor if such resistance is merely suspected, may not yet be know or even before such resistance has developed. In such cases, a co-administration, combination-therapy or treatment regime may be envisioned, by appropriate use of the subject platinum-based compounds together with the appropriate non-platinum-based therapeutic agent. In alternative embodiments of all aspects of the instant invention, the subject platinum-based compounds will be useful in treating individuals suffering from a cancer or a tumor that has been previously treated with any of the non-platinum-based therapeutic agent. In such embodiments, it may be subsequently determined, or not at all, that the cancer or tumor was resistant or refractory to the non-platinum-based therapeutic agent.

Cell lines according to the invention that may be used to evaluate whether the compounds of this invention possess cytotoxic activity against drug-resistant cell lines include but are not limited to the taxene-resistant tubulin-mutated cell lines 1A9-PTX10 and 1A9-PTX22 and their parental cell line 1A9 (J Biol Chem (1997) 272, 17118-17124), the adriamycin-resistant P-gp overexpressing cell line NCI-Adr resistant (Vickers et al., 1989. Mol Endocrinology 3 (1):157-164), the camptothecin-resistant cell lines CEM/C1 and CEM/C2 and their parental cell line CEM (Kapoor et al., 1995. Oncology Research 7; 83-95), the TWIST overexpressing cell line HNE1-T3 and its parental cell line (Oncogene (2004) 23, 474-482), the paclitaxel-resistant subclones of the human ovarian cancer cell line SKOV-3 and SKOV-3 (Clin Cancer Res (2003) 9, 2778-2785), the mitoxantrone-resistant colon cancer carcinome cell line HT29/MIT and its parental cell line HT29 (Cancer Res (2001) 61, 6034-6037), and the etoposide-resistant breast cancer cell line MCF-7/VP and its parental cell line MCF-7 (Cancer Res (1994) 54, 152-158; Int J Cancer (1997) 71, 35-41).

The subject platinum-based compounds are also believed useful in treating other types of proliferative disorders, including, proliferative disorders which are characterized by benign indications. Such disorders may also be known as "cytoproliferative" or "hyperproliferative" in that cells are made by the body at an atypically elevated rate. Such disorders include, but are not limited to, the following: hemangiomatosis in new born, secondary progressive multiple sclerosis, chronic progressive myelodegenerative disease, neurofibromatosis, ganglioneuromatosis, keloid formation, Paget's disease of the bone, fibrocystic disease of the breast, Peronies and Duputren's fibrosis, restenosis and cirrhosis.

### VII. Combination Therapy

The subject pharmaceutical compositions can be co-administered, e.g., in the same or different formulation, with a variety of other drugs. For example, the subject pharmaceutical compositions can be used as part of a regiment of treatment in which they are combined with other chemotherapeutic agents including anti-cancer therapeutic agents that inhibit cancer growth, anti-angiogenesis agents and anti-metastatic agents. The subject pharmaceutical compositions may also be combined with immunomodulators.

In a preferred embodiment, the subject pharmaceutical compositions are co-administered with an agent that tackles and/or overcomes a specific drug resistance mechanism. In this context, a "specific drug resistance mechanism" refers to any physiological or cellular mechanism which causes a cancer, tumor, cancer cell or tumor cell to become resistant to an anti-cancer therapeutic agent, but which drug resistance mechanism can be overcome, i.e. resistance is circumvented, by administering suitable compounds, agents or pharmaceutical agents. For example, when a tumor drug resistance is caused by an increase in drug efflux brought about by an overexpression of ATP-dependent pumps such as P-glycoprotein, pharmacological agents that reverse the excessive drug efflux through P-glycoprotein can be used in combination with the subject pharmaceutical compositions for treating resistance tumors. Therefore, in one embodiment, the subject pharmaceutical compositions are co-administered with an agent that overcomes a specific drug resistance mechanism. More preferably, said specific drug resistance mechanism is an increase in drug efflux brought about by ATP-binding cassette transporters. Such suitable pharmacological agents include, for example, phenothiazines, verapamil, tamoxifen, quinidine, phenothiazines, cyclosporine A, methylenedioxymethamphetamine, methylenedioxyethylamphetamine, paramethoxyamphetamine, pervilleine F, PSC 833 and LY335979, among others. For a general discussion of the pharmacologic implications for the clinical use of P-glycoprotein inhibitors, see Lum et al., Drug Resist. Clin. Onc. Hemat., 9: 319-336 (1995); Schinkel et al., Eur. J. Cancer, 31A: 1295-1298 (1995).

One particular drug that can be combined with the subject pharmaceutical compositions is PSC-833 (Valspodar), an analogue of cyclosporine. PSC-833 was found to be a P-gp inhibitor 10 times more potent than cyclosporine, and without its side-effects of nephrotoxicity and immunosuppression. Although combination phase I and II studies in different tumor types showed that PSC-833 had a profound effect on the pharmacokinetics of the co-administered chemotherapy, this effect could be adjusted for by reducing the dose of chemotherapy given. See Baird and Kaye, 2003.

In another embodiment, when a tumor drug resistance is caused by a mutation in β-tubulin, cancer therapeutic agents that have cellular targets other than microtubules may be used in combination with the subject pharmaceutical compositions.

In a further embodiment, the subject platinum-based compound is administered to a patient to whom an anti-emetic agent is also administered. Anti-emetic agents according to this invention include any anti-emetic agents known to the skill artisan, including, but not limited to, serotonin-3 receptor antagonists like granisetron, ondansetron and tropisetron, NK1 receptor antagonists, antihistamines such as cinnarizine, cyclizine and promethazine, histamine H2 receptor antagonists such as ranitidine (Zantac), phenothiazines such as chlorpromazine, droperidol, haloperidol, methotrimeprazine, perphenazine, trifluoperazine and prochlorperazine, domperidone, and metoclopramide.

In other embodiments, the subject platinum-based compound is administered to a patient who is also treated with an anti-diarrheal such as loperamid, corticosteroide such as cortisone, growth hormone or growth factor such as GCSF or erythropoietin, a diuretica such as furosemid, steroidal or non-steroidal analgesics such as an opiate, e.g. morphine, or paracetamol or anti-hyperuricemics such as allopurinol.

In other embodiments, the subject platinum-based compound is administered to a patient, who is also treated with thrombocytes, erythrocytes or whole blood.

In other embodiments, the subject platinum-based compound is administered to a patient, who is also treated with stem cells of the bone marrow.

In other embodiments, the instant invention also relate to a method of therapeutic patient care. In the method, a patient who is administered the subject platinum-based compound receives food parenterally.

The term "co-administer" or "co-administered", as used herein, include administering two or more different therapeutic agents concurrently, sequentially or intermittently in all of the various aspects of the method of the invention. Thus, the subject platinum-based compounds may be administered before, after, or together with one or more other therapeutic agents to an individual in need of. In one embodiment, two or more therapeutic agents are formulated together with the subject platinum-based compound in a single pill.

The methods of the present invention can also be combined with other methods of cancer treatment, such as radiation therapy, surgery, immunotherapy.

### VIII. Packaged Pharmaceuticals and Other Methods

The present invention also provides a packaged pharmaceutical comprising a pharmaceutical composition of the subject platinum-based compounds and instructions to administer an effective amount of the pharmaceutical composition to a cancer patient previously treated with, and preferably resistant or refractory to, a non-platinum-based chemotherapeutic agent.

In a particular embodiment, the packaged pharmaceutical further comprises another pharmaceutical ingredient and/or instructions to further administer an effective amount of another pharmaceutical ingredient. In certain embodiments said other pharmaceutical ingredient is an anti-emetic or anti-diarrheal therapeutic composition. In other embodiments said other pharmaceutical ingredient is an agent that overcomes a specific drug resistance mechanism, such as an increase in drug efflux brought about by ATP-binding cassette transporters

The present invention further provides methods of conducting a pharmaceutical business, which comprises:
a) compiling data including:
   i. bioequivalence data for a platinum chemotherapeutic compound selected from JM216, JM118, JM383 and their metabolites compared to a marketed originator compound; or
   ii. clinical data demonstrating the effectiveness of said platinum chemotherapeutic compound in treating cancer patients previously treated with a non-platinum-based chemotherapeutic agent;
b) submitting said compiled data to a drug regulatory authority for the purpose of obtaining regulatory or marketing approval for said platinum chemotherapeutic compound for the treatment of cancer patients previously treated with, or preferably resistant or refractory to, a non-platinum-based chemotherapeutic agent; and
c) preparing or proceeding to manufacture, import, package/re-package, label/re-label or market said platinum chemotherapeutic compound, or license rights to said approval, for the treatment of cancer patients previously treated with a non-platinum-based chemotherapeutic agent.

In a particular embodiment, the methods for conducting a pharmaceutical business further include marketing, distributing or selling said platinum-based compound for the treatment of cancer patients previously treated with a non-platinum-based agent.

Such methods may be followed, for example, by a generic pharmaceutical company wishing to introduce a generic version of a previously approved therapeutic compound on to the market.

### VIII. Pharmaceutical formulations

The compositions of this invention can be formulated and administered to treat individuals with cancer resistant to a non-platinum-based therapeutic agent by any means that produces contact of the active ingredient with the agent's site of action in the body of said individual, e.g. a mammal. They can be administered by any conventional means available for use in conjunction with pharmaceuticals, either as individual therapeutic active ingredients or in a combination of therapeutic active ingredients, such as by further administering another different therapeutic agent. They can be administered alone, but are generally administered with a pharmaceutical carrier selected on the basis of the chosen route of administration and standard pharmaceutical practice.

Pharmaceutical compositions for use in accordance with the present invention may be formulated in conventional manner using one or more physiologically acceptable carriers or excipients. The pharmaceutical compositions of the invention can be formulated for a variety of routes of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous (i.m., i.v., i.p., and i.c. respectively). For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

The most preferred administration route is oral. In oral administration, the pharmaceutical compositions may take the form of, for example, unit dose-forms such as tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active agent. For buccal administration the therapeutic compositions may take the form of tablets or lozenges formulated in a conventional manner. For administration by inhalation, the compositions for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the therapeutic compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the compositions of the invention are formulated into ointments, salves, gels, or creams as generally known in the art. A wash solution can be used locally to treat an injury or inflammation to accelerate healing. For oral administration, the therapeutic compositions are formulated into conventional oral administration forms such as capsules, tablets, and tonics.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydropropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

The pharmaceutical compositions of the present invention may be formulated in a neutral or salt form. Pharmaceutical-acceptable salts include the acid addition salts and are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The present invention also provides methods for formulating a pharmaceutical composition useful for the treatment of a disease selected from a cancer or tumor resistant or refractory to a non-platinum-based therapeutic agent, comprising formulating with a pharmaceutically acceptable carrier, diluent or vehicle, a platinum-based compound selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 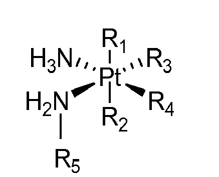 wherein R₁ and R₂ may be present or absent, each of R₁-R₄ is independently selected from halogen, hydroxyl, and acetate, and R₅ is a cycloalkyl;
(d) satraplatin or a metabolite of satraplatin; or a pharmaceutically acceptable salt, isomer or prodrug of (a) to (d).

In a preferred embodiment, the subject invention contemplates the use of a platinum-based chemotherapeutic agent selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 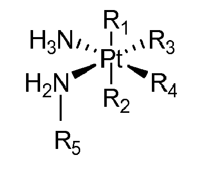 wherein R₁ and R₂ may be present or absent, each of R₁-R₄ is independently selected from halogen, hydroxyl, and acetate, and R₅ is a cycloalkyl; and
(d) satraplatin or a metabolite of satraplatin;
or a pharmaceutically acceptable salt, isomer or prodrug of (a) to (d),
for the preparation of a pharmaceutical composition for the treatment of cancer or a tumor resistant or refractory to a non-platinum based therapeutic agent.

The present invention additionally provides methods for preparing a pharamaceutical composition useful for the treatment of an individual suffering from a cancer or tumor refractory to or previously treated with a non-platinum-based agent. The methods comprise:
a) compiling data including:
   i. bioequivalence data for a platinum chemotherapeutic compound selected from the list of JM216, JM118 and JM383 or a pharmaceutically acceptable salt, isomer or prodrug thereof, compared to a marketed originator compound; or
   ii. clinical data demonstrating the effectiveness of said platinum chemotherapeutic compound in treating cancer patients previously treated with a non-platinum-based chemotherapeutic agent;
b) submitting said compiled data to a drug regulatory authority for the purpose or obtaining regulatory or marketing approval for said platinum chemotherapeutic compound for the treatment of cancer patients previously treated with a non-platinum-based chemotherapeutic agent; and
c) manufacturing, importing, packaging/re-packaging, labeling/re-labeling or marketing said platinum chemotherapeutic compound, or license rights to said approval, for the treatment of cancer patients previously treated with a non-platinum-based chemotherapeutic agent.

### Dosage

The dosage administered will be a therapeutically effective amount of the compound sufficient to result in amelioration of symptoms of the cancer or tumor and will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

Toxicity and therapeutic efficacy of pharmaceutical compositions of the present invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapeutic agents which exhibit large therapeutic indices are preferred. While therapeutic compositions that exhibit toxic side effects may be used, care should be taken to design a delivery system that targets such therapeutic agents to the site of affected tissue in order to minimize potential damage to uninfected cells and, thereby, reduce side effects.

The data obtained from cell culture assays and animal studies can be used in formulating a range of dosage for use in humans. The dosage lies preferably within a range of circulating concentrations that include the ED50 with little or no toxicity. The dosage may vary within this range depending upon the dosage form employed and the route of administration utilized. For any agents used in the method of the invention, the therapeutically effective dose can be estimated initially from cell culture assays. A dose may be formulated in animal models to achieve a circulating plasma concentration range that includes the IC50 (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the small molecule will vary, for example, depending upon the identity, size, and condition of the subject or sample being treated, further depending upon the route by which the composition is to be administered, if applicable, and the effect which the practitioner desires the therapeutic to have upon the therapeutic target of targets, such as nucleic acid or polypeptide of the invention, through with the disease causes, symptoms or effects are mediated.

Exemplary doses include milligram or microgram amounts of the small molecule per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram. A person skilled in the art will appreciate that doses can also be calculated on a body surface basis. A person of 70 kg has an approximate body surface area of 1.8 square meterdoses include milligram or microgram amounts of the small molecule per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

These methods described herein are by no means all-inclusive, and further methods to suit the specific application will be apparent to the ordinary skilled artisan. Moreover, the effective amount of the compositions can be further approximated through analogy to compounds known to exert the desired effect.

The practice of aspects of the present invention may employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986). All patents, patent applications and references cited herein are incorporated in their entirety by reference.

While the invention has been described and exemplified in sufficient detail for those skilled in this art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

It should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

Those skilled in the art will recognize, or be able to ascertain using no more than routine experimentation, many equivalents to the specific embodiments of the invention described herein. Such equivalents are intended to be encompassed by the following claims. Those skilled in the art will also recognize that all combinations of embodiments, combination of aspects or features of the claims described herein are within the scope of the invention.

### EXEMPLIFICATION

### EXAMPLE 1. Efficacy of satraplatin and its metabolites is maintained in taxane-resistant tumor cells

### A. Taxane-resistant tumor cells in which resistance is mediated through P-glycoprotein

We observed the surprising finding that subject platinum-based compounds of the invention were useful in inhibiting or killing tumour cells that were resistant to other chemotherapeutic agents, where such resistance is mediated through p-glycoprotein.

Cells expressing P-glycoprotein (NCI-Adr resistant subline) were highly resistant to Adriamycin in the absence of verapamil but remained susceptible in the presence of verapamil, an inhibitor of P-glycoprotein (relative resistance was 115-fold). Upon treatment with JM216 and JM118 no change in the relative resistance was observed (relative resistance in individual experiments ranged between 1.0 to 1.5-fold).

The P-glycoprotein expressing NCI-Adr resistant subline (Vickers et al., 1989. Mol Endocrinology 3 (1):157-164) was used. 3,000-15,000 cells/well were exposed to the test compounds for 48 hours at various concentrations in the presence and absence of verapamil at 12.5 µg/ml in order to calculate the IC50 values shown in Tables 1, and cytotoxicty was measured using the SRB assay according to Shekan et al (J Natl Cancer Inst (1990) 82, 1107-112). Briefly, cells were plated in 96 well dishes 24 hours prior to compound addition. Where noted, they were exposed to verapamil for 4 hours prior to compound addition and then throughout the treatment period (48 hours). The assay was terminated with the addition of cold TCA to a final concentration of 10% and the plates were incubated for one hour at 4 °C. The plates were then washed 5 times with water and 100 µl of a Sulforhodamine B solution (4%) was added to each well. The plate was then incubated for 10 minutes at room temperature before removal of unbound dye by washing with I % acetic acid. The bound dye was solubilized with 10 mM Trizma base and the absorbance read at OD570.

### B. Taxane-resistant tumor cells in which resistance is mediated through tubulin

We observed the surprising finding that subject platinum-based compounds of the invention were useful in inhibiting or killing tumour cells that were resistant to other chemotherapeutic agents, where such resistance is mediated through tubulin.

Tubulin-mutated cells (1A9-PTX10) were highly resistant to paclitaxel and taxotere - relative resistance in individual experiments ranged between 37 to 142-fold - yet remained susceptible to treatment with JM216, JM118 and JM383 - relative resistance in individual experiments between 0.9 to 3.9-fold. The parental non-mutated cell line, 1A9, was used as control.

The tubulin mutated, paclitaxel-resistant human ovarian carcinoma cell line 1A9-PTX10 and its parental paclitaxel-sensitive cell line 1A9 were derived from the A2780 human ovarian carcinoma cell line (J Biol Chem (1997) 272, 17118-17124). 1,000-4,000 cells/well were exposed to the test compounds for 48 hours at various concentrations in order to calculate the IC50 values shown in Table 1, as above, and cytotoxicty was measured using the SRB assay according to Shekan et al. (J Natl Cancer Inst (1990) 82, 1107-112) (see Example 1A).

**Table 1. Cellular IC50's of Satraplatin and metabolites in Paclitaxel resistant cells**

| Compound | 1A9 ("non-resistant") | 1A9-PTX10 ("resistant") | | NCI-Adr resistant +verapamil ("non-resistant") | -verapamil ("resistant") | |
|---|---|---|---|---|---|---|
| | IC50 [µM] | IC50 [µM] | RR | IC50 [µM] | IC50 [µM] | RR |
| Paclitaxel | 0.005 +/- 0.003 (2) | 0,213 +/- 0.069 (2) | 42.6 | 0.031 (1) | 1.42 (1) | 46 |
| Taxotere | 0,002 +/- 0.000 (2) | 0,212 +/- 0.104 (2) | 105.8 | 0.019 (1) | 0.62 (1) | 33 |
| Satraplatin (JM216) | 4.4 +/- 3.1 (3) | 5.7 +/- 1.6 (3) | 1.3 | 21.9 +/-0.0 (2) | 20.5 +/- 1.6 (2) | 1.1 |
| JM118 | 0.5+/-0.2(3) | 1.5+/-0.6(3) | 3.1 | 2.3+/-1.3(2) | 2.1 (1) | 1.1 |
| JM383 | 7.6 +/- 1.7 (3) | 16.0 +/- 5.7 (3) | 2.1 | | | |
| Cisplatin | 8.4 +/- 4.2 (3) | 13.9 +/- 5.2 (3) | 1.6 | 13.0 +/- 3.5 (2) | 13.2 +/- 2.6 (2) | 0.93 |
| Adriamycin | | | | 75 (1) | 0,65 (1) | 115 |

| | | | | | | |
|---|---|---|---|---|---|---|
| Legend: Table 1 shows the IC50 values as determined in the experiments described in Example 1. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e., the level of resistance conferred to the indicated drugs by the respective resistance mechanisms, and is calculated as the ratio of the means of the resistant cells compared to the sensitive cells. | | | | | | |

### EXAMPLE 2. Efficacy of satraplatin and its metabolites is maintained in campothecin-resistant tumor cells

We observed the surprising finding that subject platinum-based compounds of the invention were useful in inhibiting or killing tumour cells that were resistant to other chemotherapeutic agents, where such resistance is mediated through topoisomerase I.

Topoisomerase-mutated cells (CEM/C2) were highly resistant to camptothecin - relative resistance in individual experiments ranged between 1,280 to 1,775-fold - yet remained susceptible to treatment with JM216, JM118 and JM383 - relative resistance in individual experiments between 0.48 to 0.82-fold. The parental non-mutated cell line, CEM, was used as control.

The Camptothecin resistant CEM/C2 cells were derived from the T lymphoblastoid leukemia cell line CCRF/CEM by selection in the presence of Camptohecin *in vitro* (Kapoor et al., 1995. Oncology Research 7; 83-95, ATCC

The effects of satraplatin and metabolites were evaluated in the CCRF/CEM and CEM/C2 cells using the Calcein AM viability assay (Molecular Probes) adapted for flow cytometry. The cells were plated in T-25 flasks in the presence or absence of compound and incubated for 48 hours before termination of the assay. Cells were washed twice with PBS and incubated in 200 µl of a solution of Calcein AM (0.3 nM)/PBS for 90 minutes at 37°C. The cells were washed once with PBS before determining fluorescent units on a FACScan flow cytometer.

**Table 2. Cellular IC50's of Satraplatin and metabolites in Campothecin resistant cells**

| Compound | CEM ("non-resistant") IC50 (µM) | CEM/C2 ("resistant") IC50 (µM) | RR |
|---|---|---|---|
| Camptothecin | 0,005 +/- 0.001 (2) | 6.75 +/- 0.49 (2) | 1500 |
| Satraplatin (JM216) | 5.06 +/- 1.97 (2) | 4.05 +/- 1.79 (2) | 0.80 |
| JM118 | 0.43 +/- 0.22 (2) | 0.50 +/- 0.42 (2) | 1.18 |
| JM383 | 23.8 +/- 3.1 (2) | 12.2 +/- 0.5 (2) | 0.51 |
| Cisplatin | 2.60 +/- 1.07 (2) | 2.2 +/- 1.5 (2) | 0.85 |

| | | | |
|---|---|---|---|
| Legend: Table 2 shows the IC50 values as determined in the experiments described in Example 2. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the level of resistance conferred to the indicated drugs through atypical multi-drug resistance and mutant topoisomerase I. | | | |

### EXAMPLE 3. Efficacy of satraplatin and its metabolites is maintained in tumor cells in which resistance is mediated through ATP-binding cassette (ABC) transporters

We observed the surprising finding that subject platinum-based compounds of the invention were useful in inhibiting or killing tumour cells that were resistant to other chemotherapeutic agents, where such resistance is mediated through ATP-binding cassette (ABC) transporters.

### A. Resistance mediated through BCRP (ABCG2)

The mitoxantrone-resistant colon carcinoma cell line HT29/MIT was used (Perego et al, Cancer Res, 61, 6034). The development of drug resistance in this cell line is associated with the up-regulation of the breast carcinoma resistance protein (BCRP; ABCG2). The parental cell line, HT29, was used as a control.

2,000 cells/well were exposed to the test compounds for 48 hours at various concentrations in order to calculate the IC50 values shown in Table 3, as above, and cytotoxicty was measured using the SRB assay according to Shekan et al. (J Natl Cancer Inst (1990) 82, 1107-112) (see Example 1A). HT29/MIT cells were highly resistant to mitoxantrone - relative resistance in individual experiments ranged between 105 to 239-fold - yet remained susceptible to treatment with satraplatin and JM118 - relative resistance in individual experiments between 1.2 and 2.0-fold.

### B. Resistance mediated through MRP1 (ABCC1)

The etoposide-resistant breast cancer cell line MCF-7/VP was used (Schneider et al, Cancer Res, 54, 152). The development of drug resistance in this cell line is associated with the up-regulation of the multiple drug resistance protein 1 (MRP1; ABCC1; Schneider et al, Cancer Res, 54, 152; Perez-Soler et al, Int J Cancer, 71,35). The parental cell line, MCF-7, was used as a control.

2,000 cells/well were exposed to the test compounds for 48 hours at various concentrations in order to calculate the IC50 values shown in Table 3, as above, and cytotoxicty was measured using the SRB assay according to Shekan et al. (J Natl Cancer Inst (1990) 82, 1107-112) (see Example 1A). MCF-7/VP cells were highly resistant to etoposide - relative resistance in three individual experiments was determined to be >20-fold - yet remained susceptible to treatment with satraplatin, cisplatin and JM118 - relative resistance in individual experiments between 1.0 and 1.8-fold.

**Table 3. Cellular IC50's of Satraplatin and metabolites in mitoxantrone and etoposide resistant cells**

| Compound | HT-29 ("non-resistant") | HT-29/MIT ("resistant") | | MCF7 ("non-resistant") | MCF7/VP ("resistant") | |
|---|---|---|---|---|---|---|
| | IC50 [µM] | IC50 [µM] | RR | IC50 [µM] | IC50 [µM] | RR |
| Mitoxantrone | 1.3 +/- 0.6 (3) | 198 +/-15 (3) | 175 +/-67 | | | |
| Etoposide | | | | 7.2 +/- 2.5 (3) | >100 (3) | >20 |
| Satraplatin | 6.9 +/- 2.8 (3) | 10.2 +/- 2.2 (3) | 1.6 +/- 0.3 | 3.0 +/- 1.2 (3) | 4.8 +/- 1.4 (3) | 1.6 +/- 0.2 |
| JM118 | 4.5 +/- 2.3 (4) | 6.0 +/- 3.3 (4) | 1.3 +/- 0.1 | 1.2 +/- 0.3 (3) | 1.3 +/- 0.3 (3) | 1.1 +/- 0.1 |
| Cisplatin | | | | 28.7 +/- 3.6 (3) | 31.2 +/- 7.1 (3) | 1.1 +/- 0.1 |

Table 3 shows the IC50 values as determined in the experiments described in Example 3. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the relative level of resistance conferred to the indicated drugs by the respective resistance mechanisms.

### EXAMPLE 4. Efficacy of satraplatin and its metabolites is maintained in cisplatin-resistant tumor cells

We observed the surprising finding that subject platinum-based compounds of the invention were useful in inhibiting or killing tumour cells that were resistant to other platinum compounds, such as cisplatin.

The A129 cp80 cell line (received from Tito Fojo, NIH; Biochem Pharmacol 52, 1855), derived from the ovarian carcinoma A2780, was highly resistant to cisplatin - relative resistance in individual experiments ranged between 80 to 106-fold - yet remained susceptible to treatment with JM216, JM118 and JM383 - relative resistance in individual experiments between 0.19 to 2.59-fold (Table 1). The parental non-mutated cell line A129 was used as control.

1,000-5,000 cells/well were exposed to the test compounds for 48 hours at various concentrations in order to calculate the IC50 values shown in Tables 3. Cytotoxicty was measured using the SRB assay according to Shekan et al. as described above in Example 1A.

**Table 4. Cellular IC50's of Satraplatin and metabolites in Cisplatin resistant cells**

| Compound | Cell line | | |
|---|---|---|---|
| | A129 ("non-resistant") IC50 (µM) | A129 cp80 ("resistant") IC50 (µM) | RR |
| Cisplatin | 0.13 +/- 0.06 (2) | 11.6 +/- 3.0 (2) | 89 |
| Satraplatin (JM216) | 0.17+/-0.01 (2) | 1.13+/-0.18(2) | 6.8 |
| JM118 | 0.17 +/- 0.06 (2) | 0.43 +/- 0.33 (2) | 2.6 |
| JM383 | 1.29 +/- 0.11 (2) | 1.78 +/- 1.15 (2) | 1.4 |

| | | | |
|---|---|---|---|
| Legend: Table 4 shows the IC50 values as determined in the experiments described in Example 4. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the relative level of resistance conferred to the indicated drugs by the mechanism that confers cisplatin resistance. | | | |

### References

R.D. Baird and S.B. Kaye, Drug resistance reversal- are we getting closer?, European Journal of Cancer, 39: 2450-61 (2003).
P. Giannokakou et al., J. Biol. Chem. 272: 17118-125 (1997).
P. Giannokakou et al., Proc. Natl. Acad. Sci. U.S.A. 97: 2904-2909 (2000)
Kelland L R. An update on satraplatin: the first orally available platinum anticancer drug. Expert Opin Investig Drugs 9(6):1373-1382, 2000.

## Claims

1. A method of killing or inhibiting the growth of a tumor cell resistant to a non-platinum-based therapeutic agent comprising exposing said cell to an effective amount of a platinum-based chemotherapeutic agent selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 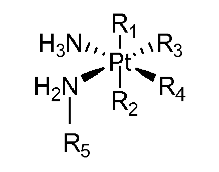 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d).

2. A method for treating an individual with a tumor resistant or refractory to a non-platinum-based therapeutic agent, comprising administering to the individual an effective amount of a platinum-based chemotherapeutic agent selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 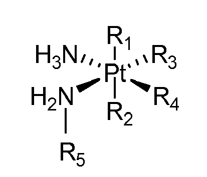 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of any of (a) to (d).

3. The method of claim 1 or 2, wherein R₅ is cyclohexyl.

4. The method of claim 1 or 2, wherein said platinum-based compound is selected from: JM216, JM118 and JM383, or a pharmaceutically acceptable salt, isomer or prodrug thereof.

5. The method of any one of claims 1-4, wherein the resistance of said tumor cell or said tumor to a non-platinum-based agent is mediated by multidrug resistance.

6. The method of claim 5, wherein the resistance of said tumor cell or said tumor to a non-platinum-based therapeutic agent is mediated through an ATP-binding cassette (ABC) transporter.

7. The method of claim 6, wherein the ATP-binding cassette transporter is P-glycoprotein (ABCB1), breast carcinoma resistance protein (ABCG2) or multiple drug resistance protein 1 (ABCC1).

8. The method of claim 7, wherein the non-platinum based therapeutic agent is selected from: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide) and anti-mitotics.

9. The method of any one of claims 1-4, wherein the resistance of said tumor cell or said tumor to a non-platinum-based therapeutic agent is mediated through tubulin.

10. The method of claim 9, wherein the non-platinum based therapeutic agent is selected from: taxanes (paclitaxel, docetaxel and taxol derivatives), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole.

11. The method of any one of claims 1-4, wherein the resistance of said tumor cell or said tumor to a non-platimum-based therapeutic agent is mediated through topoisomerase I.

12. The method of claim 11, wherein the non-platinum based therapeutic agent is selected from: camptothecin, 9-nitrocamptothecin (Orethecin, rubitecan), 9-aminocamptothecin (IDEC-13'), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D and irinotecan (Camptosar, CPT-11).

13. The method of any one of claims 1-4, wherein said tumor cell or said tumor is resistant to a non-platinum-based therapeutic agent selected from: paclitaxel, docetaxol, adriamycin, mitoxantrone, etoposide and camptothecin.

14. The method of any one of claims 1-4, wherein said tumor comprises a solid tumor or wherein said tumor cell is included in a solid tumor.

15. The method of claim 14, wherein said solid tumor is selected from: breast cancer, cervical cancer, colorectal cancer, peritoneal cancer, ovarian cancer, bronchial cancer, small cell lung cancer, non-small cell lung cancer, gastric, prostate, and head and neck cancer, or metastases thereof.

16. The method of any one of claims 1-4, wherein said tumor comprises a hematological tumor or wherein said tumor cell is included in a hematological tumor.

17. The method of any one of claims 1-4, wherein said platinum-based compound is administered to an individual diagnosed with a cancer or tumor refractory to, or previously treated with, a non-platinum-based therapeutic agent.

18. The method of claim 17, wherein said individual is further administered with one or more anti-emetic or anti-diarrheal agents.

19. The method of claim 17, wherein said individual is further treated with one or more other anti-cancer therapeutic agents.

20. The method of claim 19, wherein said other anti-cancer therapeutic agent is an agent that overcomes a specific drug resistance mechanism.

21. The method of claim 20, wherein said specific drug resistance mechanism is an increase in drug efflux brought about by ATP-binding cassette transporters.

22. The method of any one of claims 1 to 4, wherein said non-platinum-based therapeutic agent is not a hormone-based drug.

23. A method for treating an individual with a tumor resistant or refractory to paclitaxel, docetaxol, adriamycin, mitoxantrone, etoposide or camptothecin, comprising administering to the individual an effective amount of satraplatin.

24. The use of a platinum-based chemotherapeutic agent selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 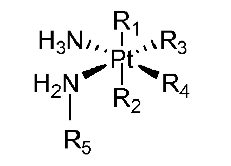 wherein R₁ and R₂ may be present or absent, each of R₁-R₄ is independently selected from halogen, hydroxyl, and acetate, and R₅ is a cycloalkyl; and
(d) satraplatin or a metabolite of satraplatin;
or a pharmaceutically acceptable salt, isomer or prodrug of (a) to (d),
for the preparation of a pharmaceutical composition for the treatment of cancer or a tumor resistant or refractory to a non-platinum based therapeutic agent.

25. The use of satraplatin for the preparation of a pharmaceutical composition for the treatment of a cancer or a tumor resistant or refractory to paclitaxel, docetaxol, adriamycin, mitoxantrone, etoposide or camptothecin.

26. A packaged pharmaceutical comprising a pharmaceutical composition of a platinum-based chemotherapeutic agent selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 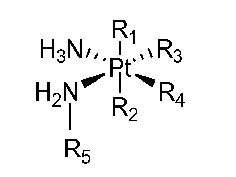 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of any of (a) to (d); and
wherein said packaged pharmaceutical further comprises instructions to administer an effective amount of the pharmaceutical composition to an individual suffering from a cancer or tumor resistant or refractory to a non-platinum-based therapeutic agent.

27. The packaged pharmaceutical of claim 26, wherein R₅ is cyclohexyl.

28. The packaged pharmaceutical of claim 26 or 27, further comprising another pharmaceutical ingredient and/or instructions to further administer an effective amount of another pharmaceutical ingredient.

29. The packaged pharmaceutical of claim 28, wherein said other pharmaceutical ingredient is an anti-emetic or anti-diarrheal therapeutic composition.

30. The packaged pharmaceutical of claim 28, wherein said other pharmaceutical ingredient is an agent that overcomes a specific drug resistance mechanism.

31. A packaged pharmaceutical comprising a pharmaceutical composition of satraplatin, wherein said packaged pharmaceutical further comprises instructions to administer an effective amount of the pharmaceutical composition to an individual suffering from a cancer or tumor resistant or refractory to paclitaxel, docetaxol, adriamycin, mitoxantrone, etoposide or camptothecin.

32. A pharmaceutical composition for use in treating a disease selected from a cancer or a tumor resistant or refractory to non-platinum-based therapeutic agent, comprising a platinum-based compound together with a pharmaceutically acceptable carrier, diluent or vehicle, wherein the platinum-based chemotherapeutic agent is selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 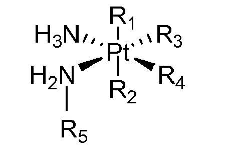 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d).

33. The pharmaceutical composition of claim 32, wherein R₅ is cyclohexyl.

34. A method for treating an individual suffering from a cancer or tumor resistant or refractory to a taxane-based therapeutic agent, comprising administering to the individual an effective amount of JM216, JM118 and JM383, or a pharmaceutically acceptable salt, isomer or prodrug thereof.

35. A method for treating an individual suffering from a cancer or tumor resistant or refractory to a camptothecin-based therapeutic agent, comprising administering to the individual an effective amount of JM216, JM118 and JM383, or a pharmaceutically acceptable salt, isomer or prodrug thereof.

36. An article of manufacture comprising a pharmaceutical composition and a label which indicates that said pharmaceutical composition can be used for the treatment of an individual suffering from a cancer or tumor resistant or refractory to a non-platinum-based chemotherapeutic agent, wherein said pharmaceutical composition comprises a platinum-based compound together with a pharmaceutically acceptable carrier, diluent or vehicle, wherein the platinum-based compound is selected from:
(a) an orally available platinum-based chemotherapeutic agent;
(b) a platinum-based chemotherapeutic agent comprising a platinum (IV) coordination complex;
(c) a platinum-based chemotherapeutic agent represented in the following general structure: 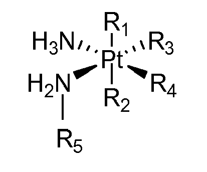 wherein R₁ and R₂ may be present or absent, each of R₁-R₄ is independently selected from halogen, hydroxyl, and acetate, and R₅ is a cycloalkyl, optionally cyclohexyl;
(d) satraplatin or a metabolite of satraplatin;
or a pharmaceutically acceptable salt, isomer or prodrug of (a) to (d).

37. The article of claim 36, further comprising packaging material, wherein said pharmaceutical composition is contained within said packaging, or wherein said label is contained in or is comprised by said packaging.

38. The article of claim 36, further comprising an anti-emetic or anti-diarrheal agent, or wherein said label further indicates that an anti-emetic or anti-diarrheal agent is to be further administered with said pharmaceutical composition.
